# EUROPÄISCHE PATENTSCHRIFT

(11) **EP 4 149 920 B1**
(45) Veröffentlichungstag und Bekanntmachung des Hinweises auf die Patenterteilung: **31.07.2024**
(21) Anmeldenummer: 21720172.2
(22) Anmeldetag: 30.03.2021
(51) Int. Cl.: C07C 263/04, C07C 265/04, C07C 265/06, C07C 265/08, C07C 265/14, C07F 7/10, C07F 7/12, C07F 7/18, C07F 7/08

(54) **VERFAHREN ZUR SYNTHESE VON ISOCYANATEN**
PROCESS FOR THE SYNTHESIS OF ISOCYANATES
PROCÉDÉ DE SYNTHÈSE D'ISOCYANATES

(30) Priorität: 13.05.2020 DE 102020113028
(43) Veröffentlichungstag der Anmeldung: 22.03.2023
(73) Patentinhaber: Technische Universität Bergakademie Freiberg, 09599 Freiberg (DE)
(72) Erfinder: KROKE, Edwin, 09633 Halsbrücke (DE); GRÜNDLER, Franziska, 09599 Freiberg (DE); HERBIG, Marcus, 09599 Freiberg (DE); BAUMHARDT, Marlene-Kirstin, 09599 Freiberg (DE)
(74) Vertreter: Riechelmann & Carlsohn Patentanwälte PartG mbB
(86) Internationale Anmeldenummer: PCT/DE2021/100312
(87) Internationale Veröffentlichungsnummer: WO 2021/228301

(56) Entgegenhaltungen:
- LEBEDEV A V ET AL: "Organosilicon synthesis of isocyanates: II. Synthesis of aliphatic, carbocyclic, and fatty-aromatic isocyanates", RUSSIAN JOURNAL OF GENERAL CHEMISTRY, NAUKA/INTERPERIODICA, MO, vol. 76, no. 3, 1 March 2006 (2006-03-01), pages 469 - 477, XP019301330, ISSN: 1608-3350
- KIRILIN ALEKSEI D ET AL: "New aspects of isocyanate synthesis with the use ofO-silylurethanes", MENDELEEV COMMUNICATIONS, vol. 27, no. 1, 2017, pages 99 - 100, XP029898187, ISSN: 0959-9436, DOI: 10.1016/J.MENCOM.2017.01.033
- XU MAOTONG ET AL: "Stoichiometric Reactions of CO 2 and Indium-Silylamides and Catalytic Synthesis of Ureas", ANGEWANDTE CHEMIE INTERNATIONAL EDITION, vol. 56, no. 45, 6 November 2017 (2017-11-06), DE, pages 14277 - 14281, XP055819912, ISSN: 1433-7851, DOI: 10.1002/anie.201708921
- XU MAOTONG ET AL: "Stoichiometric Reactions of CO 2 and Indium-Silylamides and Catalytic Synthesis of Ureas Contents (supporting Information)", ANGEWANDTE CHEMIE INTERNATIONAL EDITION, 6 November 2017 (2017-11-06), pages 1 - 76, XP055819921, Retrieved from the Internet <URL:https://onlinelibrary.wiley.com/action/downloadSupplement?doi=10.1002%2Fanie.201708921&file=anie201708921-sup-0001-misc_information.pdf> [retrieved on 20210630]
- KIRILIN A D ET AL: "The reaction of chloromethylsilanes with amines, hexamethyldisilazanes, and silyicarbamates", RUSSIAN CHEMICAL BULLETIN, 1 January 1994 (1994-01-01), pages 1703 - 1706, XP055819926, Retrieved from the Internet <URL:https://link.springer.com/content/pdf/10.1007/BF00703492.pdf> [retrieved on 20210630]
- BIRKOFER L ET AL: "Siliciumorganische Verbindungen: LXI .N-Trimethylsilyl-Carbamidsäure-Trimethylsilylester; Ein neues Silylierungsmittel", JOURNAL OF ORGANOMETALLIC CHEMISTRY, ELSEVIER, AMSTERDAM, NL, vol. 99, 1 January 1975 (1975-01-01), pages C01 - C04, XP002092915, ISSN: 0022-328X, DOI: 10.1016/S0022-328X(00)86373-1
- SZALAY R ET AL: "Preparation, crystal structure and thermal decomposition study of some trimethylsilyl esters of dicarbamic acids", JOURNAL OF ORGANOMETALLIC CHEMISTRY, ELSEVIER, AMSTERDAM, NL, vol. 510, no. 1, 21 March 1996 (1996-03-21), pages 93 - 102, XP004036155, ISSN: 0022-328X, DOI: 10.1016/0022-328X(95)05833-B
- KNAUSZ D ET AL: "TRIMETHYLSILYLATED N-ALKYL-SUBSTITUTED CARBAMATES I. PREPARATION AND SOME REACTIONS", JOURNAL OF ORGANOMETALLIC CHEMISTRY, ELSEVIER, AMSTERDAM, NL, vol. 256, no. 1, 1 January 1983 (1983-01-01), pages 11 - 21, XP001041500, ISSN: 0022-328X, DOI: 10.1016/S0022-328X(00)99291-X
- KRICHELDORF HANS R.: "Herstellung von N-Silyloxycarbonyl-aminosäure-Derivaten", SYNTHESIS, vol. 1970, no. 05, 1 January 1970 (1970-01-01), STUTTGART, DE., pages 259 - 260, XP055820528, ISSN: 0039-7881, DOI: 10.1055/s-1970-21602

## Beschreibung

Die Erfindung betrifft ein Verfahren zur Synthese von Isocyanaten, insbesondere von aliphatischen und aromatischen Isocyanaten. Sie betrifft insbesondere ein Verfahren zur Synthese von Monoisocyanaten und zur Synthese von Diisocyanaten. Sie betrifft ferner die Verwendung von Verbindungen zur Herstellung eines Isocyanates.

Isocyanate stellen einen überaus wichtigen Rohstoff in zahlreichen Branchen dar. Hauptprodukte, die aus Diisocyanaten hergestellt werden, sind Polyurethane, die als Weich- und Hartschäume, als Komponenten für Lacke und Kunststoffteile, Isolierungen etc. in vielen Bereichen zum Einsatz kommen. Aber auch Monoisocyanate stellen wichtige reaktive Zwischenprodukte dar, um beispielsweise Harnstoff-Derivate oder Carbamat-Derivate für die Wirkstoffsynthese bereitzustellen, z. B. für pharmazeutische Produkte. Industriell werden Isocyanate unter Verwendung von Phosgen, einem äußerst giftigen und reaktiven Gas, synthetisiert. Aufgrund der Toxizität erfolgt die Produktion in kostenintensiven, hermetisch abgeriegelten Anlagen, in denen das Gas nach der Synthese direkt der Verwertung zugeführt wird. Die Synthese von Isocyanaten unter Verwendung von Phosgen wird in Lebedev, A. V., et al., in "Organosilicon synthesis of isocyanates: II. Synthesis of aliphatic, carbocyclic, and fattyaromatic isocyanates", Russian Journal of General Chemistry, Bd. 76, Nr. 3, 1. März 2006, Seiten 469-477, beschrieben.

Forschungen zu Phosgen-freien Synthesewegen von Isocyanaten wurden zahlreich durchgeführt, jedoch konnte sich keine dieser Methoden für Synthesen im größeren Maßstab durchsetzen. Beispielreaktionen sind das thermische Cracking von Carbamaten und Carbamidsäurechloriden, die Übertragung von Carbonyl-Gruppen auf Amine oder die Zersetzung von Aziden. Diese Reaktionen gehen alle mit unwirtschaftlichen Nachteilen einher, z. B. hohen Temperaturen und Drücken sowie teuren Katalysatoren. Um diesen Nachteilen entgegenzuwirken, wurde die Anwendbarkeit von CO₂ als kostengünstige Carbonyl-Ressource umfangreich untersucht. Aber auch diese Umsetzungen führten nur durch den Einsatz von Katalysatoren zu einem akzeptablen Umsatz.

Die Entstehung von Isocyanaten beim Cracking von Carbamaten wurde postuliert und im Falle von *N*-silylierten (also monosilylierten) Carbamaten beschrieben. Als Ausgangsverbindung galten dabei besonders silylierte Carbamate als vielversprechend, da durch die Silylierung die für das Cracking benötigte Temperatur reduziert werden kann (G. Greber, H. R. Kricheldorf, Angew. Chem. 1968, 80, 1028). Jedoch muss für diese Synthese zunächst ein Carbamidsäurechlorid, -anhydrid oder -ester mit vergleichsweise großem Aufwand hergestellt und als Ausgangsmaterial für die N-Silylierung zur Verfügung gestellt werden. Diese Edukte können nicht durch CO₂-Insertion in Aminosilane dargestellt werden. Unter Aminosilanen werden dabei Verbindungen verstanden, die durch Silylierung einer primären Amingruppe unter Erhalt einer direkten N-Si-Bindung erhalten wurden.

Auch O-silylierte Carbamate, die nach dem erfindungsgemäßen Verfahren durch CO₂-Insertion in Aminosilane entstehen (Stufe 2 in den Schema 9), lassen sich durch Thermolyse spalten. Jedoch werden bei der thermischen Zersetzung von diesen einfach-silylierten Carbamaten keine Isocyanate, sondern die entsprechenden Harnstoffderivate gebildet (a) DE 10 2009 045 849 A1); b) C. Wiltzsch, K. Kraushaar, A. Schwarzer, E. Kroke, Z. Naturforsch. 2011, 66b, 917; c) K. Kraushaar, C. Wiltzsch, J. Wagler, U. Böhme, A. Schwarzer, G. Roewer, E. Kroke, Organometallics 2012, 31, 4779.).

Knausz et al. postulierten 1983 die Bildung eines *N*,*O*-bissilylierten Carbamats in der kondensierten Gasphase und dessen simultane Zersetzung zum Isocyanat beim Cracking einfach-silylierter aliphatischer Carbamate (D. Knausz, A. Meszticzky, L. Szakács, B. Csäkväri, K. Üjszäszy, J. Organomet. Chem. 1983, 256, 11). Das Isocyanat konnte allerdings weder nachgewiesen noch isoliert werden, da dieses anschließend mit dem ebenfalls bei der Reaktion freigesetzten Amin sofort zum entsprechenden Harnstoff-Derivat reagiert.

Weitere Ansätze beziehen sich auf trimethylsilylierte aliphatische Dicarbamate, welche jedoch auf Katalysatoren und Wasserfänger angewiesen sind (V. F. Mironov, V. D. Sheludyakov, A. D. Kirilin, Zh. Obshch. Khim. 1976, 46, 2396) oder ausschließlich Harnstoffe als Produkte erzielen (R. Szalay, Z. Böcskei, D. Knausz, C. Loväsz, K. Üjszäszy, L. Szakács, P. Sohär, J. Organomet. Chem. 1996, 510, 93). Fuchter et al. entwickelten eine Ein-Schritt-Synthese von Harnstoffderivaten unter Verwendung von aliphatischen Aminosilanen und superkritischem CO₂ (M. J. Fuchter, C. J. Smith, M. W. S. Tsang, A. Boyer, S. Säubern, J. H. Ryan, A. B. Holmes, Chem. Commun. (Cambridge, U. K.) 2008, 2152). O-Silylcarbamate entstehen als isolierbare Zwischenprodukte. Unter Betrachtung der Forschung von Knausz (1983) vermuten sie die intermediäre Bildung von Isocyanaten. Die Arbeit mit superkritischem CO₂ stellt einen entscheidenden, wirtschaftlichen Nachteil dar.

Es ist daher nach wie vor wünschenswert, einen Phosgen-freien Syntheseweg zu finden, der eine wirtschaftliche Herstellung von Isocyanaten ermöglicht. Außerdem ist Reduktion von kostenintensiven Parametern erwünscht. Insbesondere sollen hohe Ausbeuten, ein geringer Energieverbrauch und eine Verwendung von kostengünstigen Rohstoffen erreicht werden.

Aufgabe der Erfindung ist es, die Nachteile nach dem Stand der Technik zu beseitigen. Es soll insbesondere ein Verfahren zur Synthese von Isocyanaten, insbesondere von aliphatischen und aromatischen Mono-, Di- und Triisocyanaten, angegeben werden, das deren wirtschaftliche Herstellung ohne Verwendung von Phosgen ermöglicht.

Diese Aufgabe wird durch die Merkmale der Ansprüche 1 und 15 gelöst. Zweckmäßige Ausgestaltungen der Erfindungen ergeben sich aus den Merkmalen der Unteransprüche.

Nach Maßgabe der Erfindung ist ein Verfahren zur Herstellung von Isocyanaten vorgesehen, wobei
(i) eine erste siliciumorganische Verbindung, die zumindest ein Silicium-Atom Si¹ und eine daran gebundene Einheit der Formel G-I 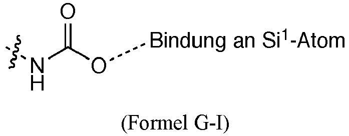 aufweist, durch Silylierung der NH-Gruppe der Einheit der Formel G-I mit einer zweiten siliciumorganischen Verbindung, die ein Silicium-Atom Si² aufweist, in eine dritte siliciumorganische Verbindung überführt wird, die eine Einheit der Formel G-II 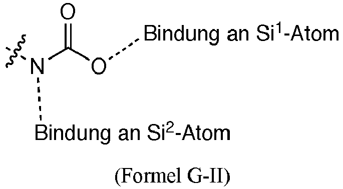 aufweist; und
(ii) die dritte siliciumorganische Verbindung durch Thermolyse zu einem Isocyanat umgesetzt wird, wobei die Einheit der Formel G-II in eine Isocyanat-Gruppe überführt wird.

Das erfindungsgemäße Verfahren ermöglicht die Herstellung von Isocyanaten, insbesondere von aliphatischen und aromatischen Isocyanaten, in hohen Ausbeuten und in hoher Reinheit. Dabei können die Isocyanate ohne Einsatz von Phosgen hergestellt werden. Das erfindungsgemäße Verfahren ermöglicht daher die sichere Herstellung von Isocyanaten. Außerdem ist das erfindungsgemäße Verfahren mit einem geringer Energieverbrauch verbunden. Es kann bei vergleichsweise moderaten Temperaturen durchgeführt werden. Der Einsatz eines Katalysators ist nicht erforderlich. Das erfindungsgemäße Verfahren kann deshalb ohne Verwendung eines Katalysators durchgeführt werden.

Das erfindungsgemäße Verfahren kann zur Herstellung von Mono-, Di- und Triisocyanaten verwendet werden. Zur Herstellung eines Diisocyanates weist die erste siliciumorganische Verbindung ein zweites Silicium-Atom Si¹ auf, an das eine zweite Einheit der Formel G-I gebunden ist. Zur Herstellung eines Triisocyanates weist die erste siliciumorganische Verbindung ein zweites Silicium-Atom Si¹, an das eine zweite Einheit der Formel G-I gebunden ist, und ein drittes Siliciumatom Si¹ auf, an das eine dritte Einheit der Formel G-I gebunden ist. Die erste siliciumorganische Verbindung kann weitere Silicium-Atome Si¹, an die jeweils eine Einheit der Formel G-I gebunden ist, aufweisen. Die Zahl der Silicium-Atome Si¹, an die jeweils eine Einheit der Formel G-I gebunden ist, entspricht der Zahl der Isocyanat-Gruppen, die das hergestellte Isocyanat aufweist. In der Einheit der Formel G-I ist ein Wasserstoffatom an das Stickstoffatom gebunden. Zur Herstellung von aliphatischen und aromatischen Isocyanaten darf kein weiteres Wasserstoffatom an dieses Stickstoffatom gebunden sein. Das gilt für aliphatische und aromatische Isocyanate unabhängig davon, ob sie substituierte oder unsubstituierte, aliphatische oder aromatische Isocyanate sind.

Die Angabe "Si¹" kennzeichnet Siliciumatome, die die erste siliciumorganische Verbindung aufweist. Die Angabe "Si²" kennzeichnet das Siliciumatom, das die zweite siliciumorganische Verbindung aufweist. Die Exponenten "1" und "2" dienen lediglich zur Kennzeichnung der Zugehörigkeit der Siliciumatome zu den jeweiligen Formeln G-I und G-II. Bei der Umsetzung der ersten siliciumorganischen Verbindung mit der zweiten siliciumorganischen Verbindung wird die dritte siliciumorganische Verbindung erhalten, die sowohl die Siliciumatome Si¹, die aus der ersten siliciumorganischen Verbindung stammen, als auch die Siliciumatome Si², die aus der zweiten siliciumorganischen Verbindung stammen, enthält.

Erfindungsgemäß kann vorgesehen sein, dass die erste siliciumorganische Verbindung eine Verbindung der allgemeinen Formel II 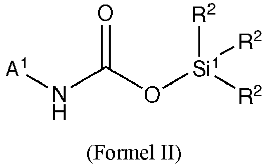 ist, worin
A¹ R¹ oder eine Gruppe der allgemeinen Formel G-III ist: 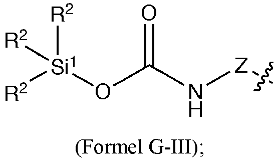
R¹ eine substituierte oder unsubstituierte, aliphatische oder aromatische Gruppe ist;
R² bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si¹ zumindest ein Rest R² gebunden ist, der nicht Wasserstoff ist; und
Z eine substituierte oder unsubstituierte, aliphatische oder aromatische Gruppe ist.

Die erste siliciumorganische Verbindung ist ein *O*-silyliertes Carbamat. Die Verbindung der allgemeinen Formel II ist ein allgemeines Beispiel eines *O*-silylierten Carbamats. Die Verbindung der allgemeinen Formel II ist eine Verbindung der allgemeinen Formel II-A, wenn A¹ R¹ ist. Die Verbindung der allgemeinen Formel II ist eine Verbindung der allgemeinen Formel II-B, wenn A¹ eine Gruppe G-III ist. 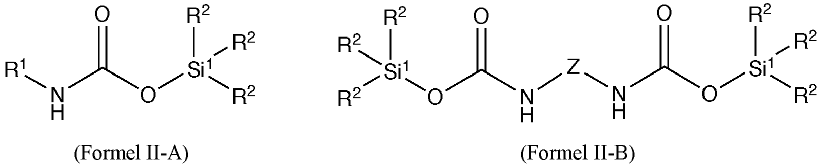 In Formel II-A haben R¹ und R² die im Zusammenhang mit der Formel II angegebenen Bedeutungen. In Formel II-B haben R² und Z die im Zusammenhang mit der Formel II angegebenen Bedeutungen. Verbindungen der allgemeinen Formel II-A sind zur Herstellung von Monoisocyanaten geeignet, Verbindungen der allgemeinen Formel II-B sind zur Herstellung von Diisocyanaten geeignet.

R¹ ist vorzugsweise eine substituierte oder unsubstituierte, aliphatische Gruppe mit 1 bis 18 Kohlenstoffatomen oder eine substituierte oder unsubstituierte aromatische Gruppe mit 6 bis 18 Kohlenstoffatomen. Ist R¹ eine aliphatische Gruppe, so kann vorgesehen sein, dass sie eine verzweigte oder unverzweigte Gruppe ist. R¹ kann eine substituierte oder unsubstituierte aliphatische Gruppe mit 1 bis 18 Kohlenstoffatomen oder mit 1 bis 12 Kohlenstoffatomen sein.

Es kann vorgesehen sein, dass R¹ aus der Gruppe ausgewählt ist, die aus einer substituierten oder unsubstituierten Alkylgruppe mit 1 bis 18 Kohlenstoffatomen, einer substituierten oder unsubstituierten Heteroalkylgruppe mit 1 bis 18 Kohlenstoffatomen, einer substituierten oder unsubstituierten Alkenylgruppe mit 1 bis 18 Kohlenstoffatomen, einer substituierten oder unsubstituierten Alkinylgruppe mit 1 bis 18 Kohlenstoffatomen, einer substituierte oder unsubstituierten Cycloalkylgruppe, einer substituierte oder unsubstituierte Heterocycloalkylgruppe, einer substituierten oder unsubstituierten Arylgruppe, einer substituierten oder unsubstituierten Heteroarylgruppe, einer substituierte oder unsubstituierten Alkylarylgruppe und einer substituierten oder unsubstituierten Arylalkylgruppe besteht. Stärker bevorzugt ist R¹ aus der Gruppe ausgewählt, die aus einer substituierten oder unsubstituierten Alkylgruppe mit 1 bis 12 Kohlenstoffatomen, einer substituierten oder unsubstituierten Alkenylgruppe mit 1 bis 12 Kohlenstoffatomen, einer substituierten oder unsubstituierten Arylgruppe, einer substituierten oder unsubstituierten Heteroarylgruppe, einer substituierten oder unsubstituierten Alkylarylgruppe und einer substituierten oder unsubstituierten Arylalkylgruppe besteht. Ist R¹ eine Alkygruppe, so kann R¹ beispielsweise aus der Gruppe ausgewählt sein, die aus einer substituierten oder unsubstituierten Methyl-Gruppe, einer substituierten oder unsubstituierten Ethyl-Gruppe, einer substituierten oder unsubstituierten Propyl-Gruppe, einer substituierten oder unsubstituierten Butyl-Gruppe, einer substituierten oder unsubstituierten Pentyl-Gruppe, einer substituierten oder unsubstituierten Hexyl-Gruppe und einer substituierten oder unsubstituierten Octyl-Gruppe besteht. Ist R¹ eine Alkenylgruppe, so kann R¹ beispielsweise eine substituierte oder unsubstituierte Allylgruppe sein. Ist R¹ eine Arylgruppe, so kann R¹ beispielsweise eine substituierte oder unsubstituierte Phenyl-Gruppe sein. Ist R¹ eine Arylalkylgruppe, so kann R¹ beispielsweise eine substituierte oder unsubstituierte Benzyl-Gruppe sein. Beispielsweise kann R¹ aus der Gruppe ausgewählt sein, die aus *n*-Butyl, *n*-Octyl, Allyl, Phenyl und Benzyl besteht. In einem anderen Beispiel ist R¹ Isopentyl.

Z ist vorzugsweise eine substituierte oder unsubstituierte, aliphatische Gruppe mit 1 bis 18 Kohlenstoffatomen oder eine substituierte oder unsubstituierte aromatische Gruppe mit 6 bis 18 Kohlenstoffatomen. Ist Z eine aliphatische Gruppe, so kann vorgesehen sein, dass sie eine verzweigte oder unverzweigte Gruppe ist. Z kann eine substituierte oder unsubstituierte aliphatische Gruppe mit 1 bis 18 Kohlenstoffatomen oder mit 1 bis 12 Kohlenstoffatomen sein.

Z ist im Gegensatz zu R¹, das eine einwertige Gruppe ist, eine zweiwertige Gruppe. Es kann vorgesehen sein, dass Z eine substituierte oder unsubstituierte Gruppe ist, die zumindest eine substituierte oder unsubstituierte Alkylengruppe und/oder zumindest eine substituierte oder unsubstituierte Arylen- oder Heteroarylengruppe aufweist. Beispielsweise kann Z aus einer Gruppe ausgewählt sein, die aus einer substituierten oder unsubstituierten Alkylengruppe mit 1 bis 18 Kohlenstoffatomen, einer substituierter oder unsubstituierten Phenylengruppe und einer substituierten oder unsubstituierten Phenylenbisalkylengruppe, in der jede Alkylengruppe bei jedem Auftreten unabhängig voneinander jeweils eine Alkylengruppe mit 1 bis 12 Kohlenstoffatomen ist, besteht.

Es ist vorgesehen, dass an jedes Siliciumatom Si¹ zumindest ein Rest R² gebunden ist, der nicht Wasserstoff ist. Damit ist ein -Si¹H₃ ausgeschlossen. Vorzugsweise sind in der Verbindung der allgemeinen Formel II R² bei jedem Auftreten unabhängig voneinander jeweils eine substituierte oder unsubstituierten Alkylgruppe mit 1 bis 6 Kohlenstoffatomen. Stärker bevorzugt ist in der Verbindung der allgemeinen Formel II R² bei jedem Auftreten unabhängig voneinander jeweils aus der Gruppe ausgewählt, die aus einer substituierten oder unsubstituierten Methyl-Gruppe, einer substituierten oder unsubstituierten Ethyl-Gruppe, einer substituierten oder unsubstituierten Propyl-Gruppe und einer substituierten oder unsubstituierten Butyl-Gruppe besteht. Noch stärker bevorzugt sind in der Verbindung der allgemeinen Formel II R² bei jedem Auftreten unabhängig voneinander jeweils aus der Gruppe ausgewählt, die aus einer unsubstituierten Methyl-Gruppe, einer unsubstituierten Ethyl-Gruppe, einer unsubstituierten Propyl-Gruppe und einer unsubstituierten Butyl-Gruppe besteht, wobei eine Methyl-gruppe besonders bevorzugt ist. In der Verbindung der allgemeinen Formel II können R² bei jedem Auftreten die gleiche Bedeutung oder unterschiedliche Bedeutungen aufweisen. Vorzugsweise sind alle R² eine Methylgruppe.

Bei der zweiten siliciumorganischen Verbindung handelt es sich um ein Silylierungsmittel. Erfindungsgemäß kann vorgesehen sein, dass die zweite siliciumorganische Verbindung eine Verbindung der allgemeinen Formel III 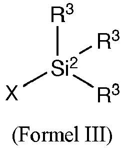 ist, worin
X aus der Gruppe ausgewählt ist, die aus einem Halogen, -CN, -OCN, -SCN, -N₃, einem Sulfonat, einem Carbamat, -O-R⁴, -NR⁷R⁸ und einem N-Heterocyclus besteht;
R³ bei jedem Auftreten unabhängig voneinander jeweils aus der Gruppe ausgewählt ist, die aus Wasserstoff einer substituierten oder unsubstituierten Alkylgruppe mit 1 bis 12 Kohlenstoffatomen und einer substituierten oder unsubstituierten Arylgruppe besteht, mit der Maßgabe, dass an jedes Silicium-atom Si² zumindest ein Rest R³ gebunden ist, der nicht Wasserstoff ist;
R⁴ -C(O)R⁹ oder eine Gruppe der allgemeinen Formel G-VI 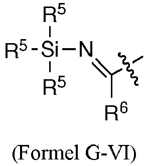 ist,
R⁵ bei jedem Auftreten unabhängig voneinander jeweils eine substituierte oder unsubstituierte Alkylgruppe mit 1 bis 12 Kohlenstoffatomen ist;
R⁶ eine substituierte oder unsubstituierte Alkylgruppe mit 1 bis 12 Kohlenstoffatomen ist;
R⁷ und R⁸ unabhängig voneinander jeweils eine substituierte oder unsubstituierte Alkylgruppe mit 1 bis 12 Kohlenstoffatomen oder -C(O)R⁹ sind; und
R⁹ eine substituierte oder unsubstituierte Alkylgruppe mit 1 bis 12 Kohlenstoffatomen ist.

Es können jedoch auch andere Silylierungsmittel eingesetzt werden. Beispiele anderer Silylierungsmittel umfassen, ohne darauf beschränkt zu sein, Aminosilane, die eine Transaminierung oder eine Transsilylierung ermöglichen, und Silazane. Siehe beispielsweise P. L. Fuchs (Hrsg.) Handbook of reagents for organic synthesis, John Wiley & Sons Inc, London, 2011**.** Weitere Beispiele geeigneter Silylierungsmittel sind in Tabelle 1 angegeben.

Die zweite siliciumorganische Verbindung trägt eine Abgangsgruppe X. Sie dient als Silylierungsmittel. Die Verbindung der allgemeinen Formel III ist ein allgemeines Beispiel eines solchen Silans.

X kann aus der Gruppe ausgewählt sein, die aus einem Halogen, einem Sulfonat, einem Carbamat, -O-R⁴, -NR⁷R⁸ und einem N-Heterocyclus besteht. Es kann vorgesehen sein, dass X ein Sulfonat ist. Unter dem Begriff "Sulfonat" wird eine R^{S}-SO₂-O-Gruppe verstanden. R^{S} kann beispielsweise eine verzweigte oder unverzweigte substituierte oder unsubstituierte C₁-C₆-Alkylgruppe, eine substituierte oder unsubstituierte Arylgruppe, eine substituierte oder unsubstituierte Alkylarylgruppe oder eine substituierte oder unsubstituierte Silangruppe sein. Vorzugsweise ist R^{S} CH₃-, CF₃-, CH₃-C₆H₄- oder -O-Si(CH₃)₃. Das Sulfonat kann beispielsweise aus der Gruppe ausgewählt sein, die aus einer Toluolsulfonsäureester-Gruppe, einer Methylsulfonsäureester-Gruppe und einer Trifluormethylsulfonsäureester-Gruppe besteht. Unter einer Toluolsulfonsäureester-Gruppe wird eine -OTs-Gruppe verstanden, wobei Ts Tosyl ist. Unter einer Methylsulfonsäureester-Gruppe wird eine -OMs-Gruppe verstanden, wobei Ms Mesyl ist. Unter einer Trifluormethylsulfonsäureester-Gruppe wird CF₃-SO₂-O- verstanden. Eine Trifluormethylsulfonsäureester-Gruppe wird auch als Triflat bezeichnet.

Es kann vorgesehen sein, dass X ein Carbamat ist. Unter dem Begriff "Carbamat" wird eine (R^{N})₂N-CO-O-Gruppe verstanden. R^{N} kann beispielsweise eine verzweigte oder unverzweigte substituierte oder unsubstituierte C₁-C₆-Alkylgruppe, eine substituierte oder unsubstituierte Arylgruppe oder eine substituierte oder unsubstituierte Alkylarylgruppe sein. Vorzugsweise ist R^{N} CH₃- oder CH₃-CH₂-. Ein Beispiel einer Verbindung der allgemeinen Formel III, in der X ein Carbamat ist, ist (CH₃)₃Si-O-CO-N(CH₃)₂.

Es kann vorgesehen sein, dass X -NR⁷R⁸ ist. In einer Variante sind R⁷ und R⁸ unabhängig voneinander jeweils eine substituierte oder unsubstituierte Alkylgruppe mit 1 bis 6 Kohlenstoffatomen, beispielsweise Methyl oder Ethyl. In einer anderen Variante ist R⁷ eine substituierte oder unsubstituierte Alkylgruppe mit 1 bis 6 Kohlenstoffatomen und R⁸ -C(O)R⁹, wobei R⁹ bevorzugt eine substituierte oder unsubstituierte Alkylgruppe mit 1 bis 6 Kohlenstoffatomen ist. Beispielsweise sind R⁷ Methyl und R⁸ -COCF₃, -COCH₃ oder -COCF₂CF₂CF₃.

Es kann vorgesehen sein, dass X ein N-Heterocyclus ist. Der Begriff "N-Heterocyclus" beschreibt eine Heterocycloalkyl-Gruppe, wie hierin definiert, oder eine Heteroarylgruppe, wie hierin definiert, jeweils mit der Maßgabe, dass zumindest eines der Heteroatome Stickstoff ist.

In der Verbindung der allgemeinen Formel III ist X vorzugsweise aus der Gruppe ausgewählt, die aus Chlor, einer Toluolsulfonsäureester-Gruppe, einer Methylsulfonsäureester-Gruppe und einer Trifluormethylsulfonsäureester-Gruppe besteht. Bevorzugt ist X eine Trifluormethylsulfonsäureester-Gruppe (Triflat).

Es ist vorgesehen, dass an jedes Siliciumatom Si² zumindest ein Rest R³ gebunden ist, der nicht Wasserstoff ist. Damit ist ein X-Si²H₃ ausgeschlossen. Vorzugsweise ist in der Verbindung der allgemeinen Formel III R³ bei jedem Auftreten unabhängig voneinander jeweils eine substituierte oder unsubstituierte Alkylgruppe mit 1 bis 6 Kohlenstoffatomen oder Phenyl. Stärker bevorzugt ist in der Verbindung der allgemeinen Formel III R³ bei jedem Auftreten unabhängig voneinander jeweils aus der Gruppe ausgewählt, die aus einer substituierten oder unsubstituierten Methyl-Gruppe, einer substituierten oder unsubstituierten Ethyl-Gruppe, einer substituierten oder unsubstituierten Propyl-Gruppe und einer substituierten oder unsubstituierten Phenyl-Gruppe besteht. Noch stärker bevorzugt ist in der Verbindung der allgemeinen Formel III R³ bei jedem Auftreten unabhängig voneinander jeweils aus der Gruppe ausgewählt, die aus einer unsubstituierten Methyl-Gruppe, einer unsubstituierten Ethyl-Gruppe, einer unsubstituierten Propyl-Gruppe und einer unsubstituierten Phenyl-Gruppe besteht, wobei eine Methylgruppe besonders bevorzugt ist. In der Verbindung der allgemeinen Formel III kann R³ bei jedem Auftreten die gleiche Bedeutung oder unterschiedliche Bedeutungen aufweisen. Vorzugsweise sind alle R³ eine Methylgruppe. Eine besonders bevorzugte Verbindung der allgemeinen Formel III ist Trimethylsilyl-trifluormethansulfonat, das auch als Trimethylsilyltriflat bezeichnet wird.

Ist X -O-R⁴ und ist R⁴ eine Gruppe der allgemeinen Formel G-VI, so kann vorgesehen sein, dass R⁵ bei jedem Auftreten unabhängig voneinander jeweils eine unsubstituierte Alkylgruppe mit 1 bis 6 Kohlenstoffatomen ist und dass R⁶ eine fluorierte oder unsubstituierte Alkylgruppe mit 1 bis 6 Kohlenstoffatomen ist. Beispielsweise können alle R⁵ und R⁶ jeweils eine Methylgruppe sein. In diesem Fall ist das Silylierungsmittel N,O-Bis(trimethylsilyl)acetamid (BSA).

Ist X -O-R⁴ und ist R⁴ -C(O)R⁹, so kann vorgesehen sein, dass R⁹ eine substituierte oder unsubstituierte Alkylgruppe mit 1 bis 6 Kohlenstoffatomen ist. Ein Beispiel einer solchen Verbindung der allgemeinen Formel III ist (CH₃)₃Si-O-CO-CCl₃.

Tabelle 1 zeigt weitere Beispiele geeigneter Silylierungsmittel.

**Tabelle 1**

| Nr. | Formel | Name (Abkürzung) |
|---|---|---|
| S1 | 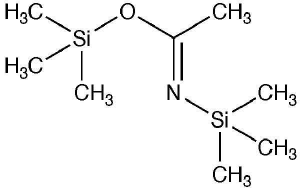 | *N,O*-Bis(trimethylsilyl)-acetamid (BSA) |
| S2 | 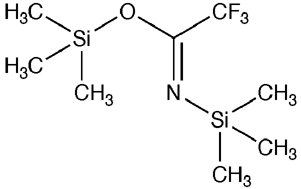 | *N*,*O*-Bis(trimethylsilyl)-trifluoracetamid (BSTFA) |
| S3 | 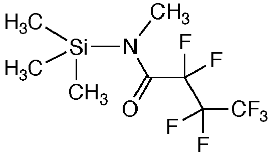 | *N*-Methyl-*N*-trimethylsilylheptafluorbutyramid (MSHFBA) |
| S4 | 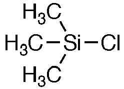 | Trimethylchlorsilan (TMCS) |
| S5 | 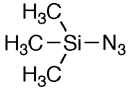 | Trimethylsilylazid (TMSA) |
| S6 | 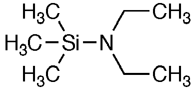 | *N*-(Trimethylsilyl)diethylamin (TMSDEA) |
| S7 | 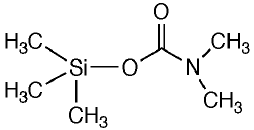 | Trimethylsilyl-*N*,*N*-dimethylcarbamat (DMCTMS) |
| S8 | 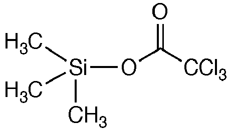 | Trimethylsilyltrichloracetat (TMSTCA) |
| S9 | 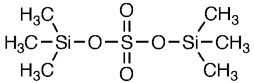 | *O,O*'-Bis(trimethylsilyl)sulfat (BSS) |
| S10 | 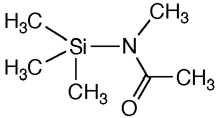 | *N*-Methyl-*N*-trimethylsilyl-acetamid (MSA) |
| S11 | 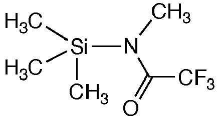 | *N*-Methyl-*N*-trimethylsilyl-trifluoracetamid (MSTFA) |
| S12 | 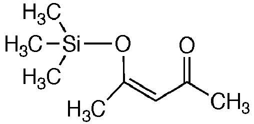 | 4-Trimethylsiloxy-3 -penten-2-on (TMSacac) |
| S13 | 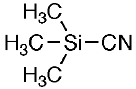 | Trimethylsilylcyanid (TMSCN) |
| S14 | 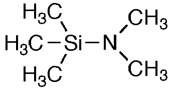 | *N*-(Trimethylsilyl)-dimethylamin (TMSDMA) |
| S15 | 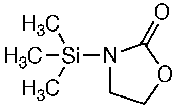 | 3-Trimethylsilyl-2-oxazolidinon (TMSO) |
| S16 | 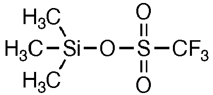 | Trimethylsilyltriflat (TMSTF) |

Es kann vorgesehen sein, dass die dritte siliciumorganische Verbindung eine Verbindung der allgemeinen Formel IV 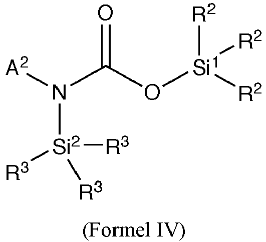 ist, worin
A² R¹ oder eine Gruppe der allgemeinen Formel G-IV ist: 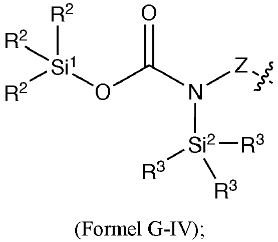
R¹, R², R³ und Z die in Zusammenhang mit der Verbindung der allgemeinen Formel II angegebenen Bedeutungen haben; und
R³ bei jedem Auftreten die in Zusammenhang mit der Verbindung der allgemeinen Formel III angegebenen Bedeutungen hat.

Die Verbindung der allgemeinen Formel IV-A ist eine Verbindung der allgemeinen Formel IV, wenn A² R¹ ist. Die Verbindung der allgemeinen Formel IV-B ist eine Verbindung der allgemeinen Formel IV, wenn A eine Gruppe G-IV ist.

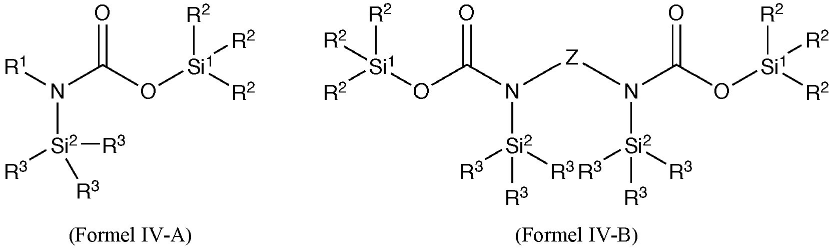

In Formel IV-A haben R¹, R² und R³ die im Zusammenhang mit der Formel II angegebenen Bedeutungen. In Formel IV-B haben R², R³ und Z die im Zusammenhang mit der Formel II angegebenen Bedeutungen. In den Formeln IV-A und IV-B haben R³ bei jedem Auftreten die in Zusammenhang mit der Verbindung der allgemeinen Formel III angegebenen Bedeutungen.

Eine Verbindung der allgemeinen Formel IV-A ist ein *N,O*-bissilyliertes Carbamat. Eine solche Verbindung ist insbesondere zur Herstellung von Monoisocyanaten geeignet. Beispiele eines *N*,*O-*bissilylierten Carbamats sind *N,O*-Bis(trimethylsilyl)-N-phenyl-carbamat (**1**), *N,O*-Bis(trimethylsilyl)-*N,n*-octyl-carbamat (**2**) und *N,O*-Bis(trimethylsilyl)-N-benzyl-carbamat (**5**). Eine Verbindung der allgemeinen Formel IV-B weist zwei *N,O*-bissilylierte Carbamat-Gruppen auf. Eine solche Verbindung ist insbesondere zur Herstellung von Diisocyanaten geeignet. Beispiele einer Verbindung mit zwei *N*,*O*-bissilylierten Carbamat-Gruppen sind *p*-[*N*,*N*',*O*,*O*'-Tetrakis(trimethylsily1)-carbamato]-benzen (**3**), *m*-[*N*,*N*',*O*,*O*'-Tetrakis(trimethylsilyl)-carbamato]-xylen (**4**) und 1,8-[*N*,*N*',*O*,*O*'-Tetrakis(trimethylsilyl)-carbamato]-octan (**6**). Die genannten Verbindungen sind in Tabelle 2 gezeigt:

**Tabelle 2**

| Nr. | Formel | Name |
|---|---|---|
| **1** | 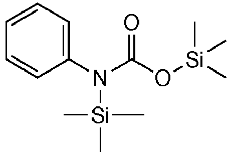 | *N*,*O*-Bis(trimethylsilyl)-*N-*phenyl-carbamat (R¹ = Phenyl) |
| **2** | 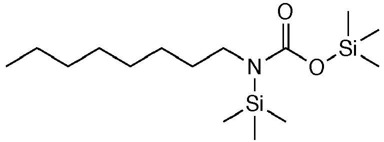 | *N,O*-Bis(trimethylsilyl)-*N,n-*octyl-carbamat (R¹ = *n*-Octyl) |
| **3** | 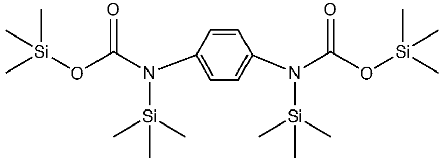 | *p*-[*N,N',O,O'-*Tetrakis(trimethylsilyl)-carbamato]-benzen (*Z* = *p*-Phenylen) |
| **4** | 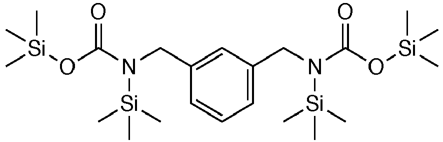 | *m*-[*N,N',O,O'-*Tetrakis(trimethylsilyl)-carbamato]-xylen (*Z* = *m*-Xylylen)) |
| **5** | 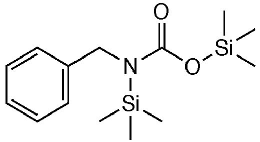 | *N*,*O*-Bis(trimethylsilyl)-*N-*benzyl-carbamat (R¹ = Benzyl) |
| **6** | 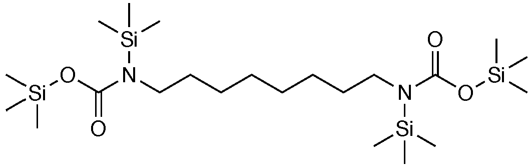 | 1,8-[*N*,*N*',*O*,*O*'-Tetrakis(trimethylsilyl)-carbamato]-octan (*Z* = -(CH₂)₈-) |

Die Verbindung der allgemeinen Formel IV kann durch Umsetzen der Verbindung der allgemeinen Formel II mit der Verbindung der allgemeinen Formel III erhalten werden. Diese Umsetzung ist eine Silylierung. Mittels der Silylierung wird eine zweite Silylgruppe in eine Carbamatgruppe eingeführt. Die zweite Silylgruppe ist an das Stickstoffatom der Carbamatgruppe gebunden, während die erste Silylgruppe an ein Sauerstoffatom der Carbamatgruppe gebunden ist. Die Verbindung der allgemeinen Formel IV-A kann durch Umsetzen der Verbindung der allgemeinen Formel II-A mit der Verbindung der allgemeinen Formel III erhalten werden. Die Verbindung der allgemeinen Formel IV-B kann durch Umsetzen der Verbindung der allgemeinen Formel II-B mit der Verbindung der allgemeinen Formel III erhalten werden.

Das erfindungsgemäße Verfahren ermöglich die Herstellung von Isocyanaten. Isocyanate werden in der vorliegenden Erfindung auch als Isocyanat-Verbindungen bezeichnet. Bei einem Isocyanat kann es sich um eine Verbindung der allgemeinen Formel I

A³-N=C=O (Formel I)

handeln, worin
A³ R¹ oder eine Gruppe der Formel G-V ist: 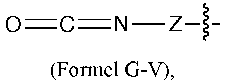
R¹ und Z die in Zusammenhang mit der Verbindung der allgemeinen Formel II angegebenen Bedeutungen haben.

Die Verbindung der allgemeinen Formel I ist eine Verbindung der allgemeinen Formel I-A, wenn A³ R¹ ist. Die Verbindung der allgemeinen Formel I ist eine Verbindung der allgemeinen Formel I-B, wenn A³ eine Gruppe G-V ist.

R¹-N=C=O (Formel I-A)

O=C=N-Z-N=C=O (Formel I-B)

In Formel I-A hat R¹ die im Zusammenhang mit der Formel II angegebenen Bedeutungen. Ist R¹ Octyl, so ist die Verbindung der allgemeinen Formel I Octylisocyanat, ist R¹ Phenyl, so ist die Verbindung der allgemeinen Formel I Phenylisocyanat, ist R¹ Benzyl, so ist die Verbindung der allgemeinen Formel I Benzylisocyanat. In Formel I-B hat Z die im Zusammenhang mit der Formel II angegebenen Bedeutungen. Ist Z Phenylen, so ist die Verbindung der allgemeinen Formel I ein Diisocyanatobenzen, beispielsweise ein 1,4-Diisocyanatobenzen. Ist Z Xylylen, so ist die Verbindung der allgemeinen Formel I ein Diisocyanatoxylylen, beispielsweise ein 1,3-Xylylendiisocyanat (Abkürzung mXDI).

Die Verbindung der allgemeinen Formel IV kann durch Thermolyse in die Verbindung der allgemeinen Formel I überführt werden. Insbesondere kann eine Verbindung der allgemeinen Formel IV-A durch Thermolyse in die Verbindung der allgemeinen Formel I-A umgesetzt werden. Eine Verbindung der allgemeinen Formel IV-B kann durch Thermolyse in die Verbindung der allgemeinen Formel I-B umgesetzt werden.

Schema 1 veranschaulicht die Herstellung eines Isocyanates durch Silylierung der ersten siliciumorganischen Verbindung, die eine Einheit G-I aufweist, unter Erhalt der dritten siliciumorganischen Verbindung, die eine Einheit G-II aufweist, und die anschließende Umsetzung der dritten siliciumorganischen Verbindung zu einem Isocyanat. 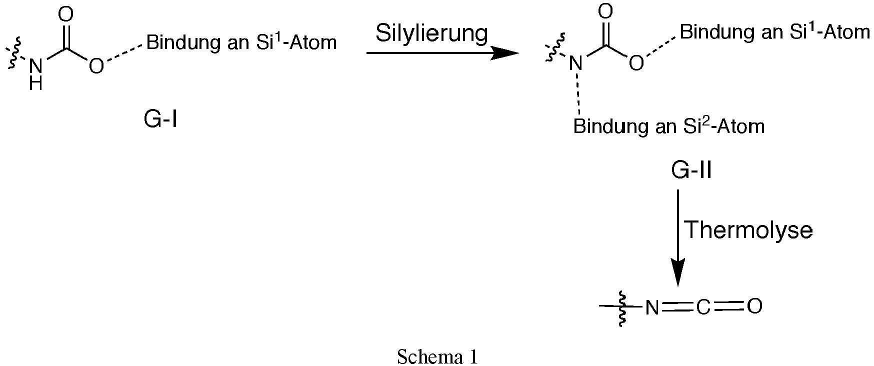 Die Silylierung wird durch Umsetzen der ersten siliciumorganischen Verbindung mit der zweiten siliciumorganischen Verbindung vorgenommen. Die beschriebene Silylierung eignet sich beispielsweise besonders zur Silylierung aromatischer O-trimethylsilylierter Carbamate, wodurch aromatische *N*,*O-*bistrimethylsilylierte Carbamate erhalten werden. Die Silylierung eignet sich aber auch zur Silylierung beispielsweise aliphatischer O-trimethylsilylierter Carbamate, wodurch aliphatische *N*,*O*-bistrimethylsilylierte Carbamate erhalten werden.

Schema 2 veranschaulicht die Herstellung eines Isocyanates der allgemeinen Formel I. Dazu wird eine Silylierung einer Verbindung der allgemeinen Formel II mit einer Verbindung der allgemeinen Formel III unter Erhalt einer Verbindung der allgemeinen Formel IV vorgenommen. Die Verbindung der allgemeinen Formel IV wird anschließend einer Thermolyse unter Erhalt des Isocyanates der allgemeinen Formel I unterzogen. 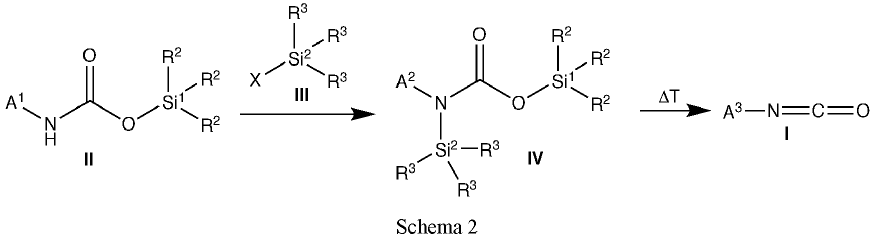 Die Angabe "ΔT" steht für Anwendung einer erhöhten Temperatur bei der Thermolyse.

Schema 3 veranschaulicht die Herstellung eines Isocyanates der allgemeinen Formel I-A. Dazu wird eine Silylierung einer Verbindung der allgemeinen Formel II-A mit einer Verbindung der allgemeinen Formel III unter Erhalt einer Verbindung der allgemeinen Formel IV-A vorgenommen. Die Verbindung der allgemeinen Formel IV-A wird anschließend einer Thermolyse unter Erhalt des Isocyanates der allgemeinen Formel I-A unterzogen.

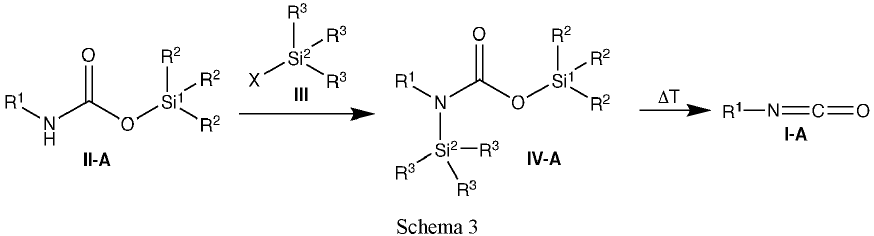

Schema 4 veranschaulicht die Herstellung eines Isocyanates der allgemeinen Formel I-B. Dazu wird eine Silylierung einer Verbindung der allgemeinen Formel II-B mit einer Verbindung der allgemeinen Formel III unter Erhalt einer Verbindung der allgemeinen Formel IV-B vorgenommen. Die Verbindung der allgemeinen Formel IV-B wird anschließend einer Thermolyse unter Erhalt des Isocyanates der allgemeinen Formel I-B unterzogen.

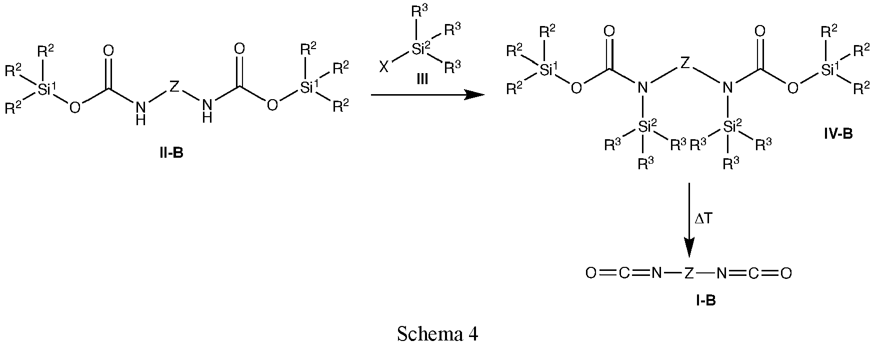

Die Silylierung der ersten siliciumorganischen Verbindung, insbesondere der Verbindung der allgemeinen Formel II, wird vorzugsweise in Gegenwart einer Hilfsbase durchgeführt. Die Hilfsbase dient zur Bindung der bei der Silylierung von der zweiten siliciumorganischen Verbindung, d. h. dem Silylierungsmittel, abgespaltenen Abgangsgruppe sowie des Wasserstoffatoms, das von der NH-Gruppe der Verbindung der allgemeinen Formel II abgespalten wird. Diese Gruppe ist die Gruppe X in der Verbindung der allgemeinen Formel III. Bei der Hilfsbase handelt es sich vorzugsweise um ein Amin, beispielsweise um ein Trialkylamin, wie Triethylamin, oder Anilin. Eine bevorzugte Hilfsbase ist ein Triethylamin. Es kann vorgesehen sein, dass zur Herstellung einer Verbindung der allgemeinen Formel I eine Hilfsbase eingesetzt wird, deren aliphatischer oder aromatischer Rest dem aliphatischen bzw. aromatischen Rest der Verbindung der allgemeinen Formel I entspricht. Beispielsweise kann zur Herstellung einer Verbindung der allgemeinen Formel I-A eine Verbindung der allgemeinen Formel VI-A als Hilfsbase eingesetzt werden. Zur Herstellung von Benzyl-isocyanat ist somit Benzylamin eine geeignete Hilfsbase, für die Herstellung von Phenylisocyanat Anilin. Ohne den Einsatz einer Hilfsbase kann die Gefahr bestehen, dass die Abgangsgruppe eine reaktive Säure bildet, die die Bildung der dritten silciumorganischen Verbindung, insbesondere der Verbindung der allgemeinen Formel IV, beeinträchtigt. Handelt es sich bei dem Silylierungsmittel um Trimethylchlorsilan und bei der Hilfsbase um Triethylamin, wird bei der Silylierung Triethylaminhydrochlorid ((CH₃CH₂)₃N*HCl) gebildet.

Die Silylierung wird vorzugsweise in einem aprotischen organischen Lösungsmittel durchgeführt. Besonders bevorzugt sind unpolare Lösungsmittel. Beispiele geeigneter Lösungsmittel sind n-Pentan, n-Hexan und Anisol, wobei n-Pentan besonders bevorzugt ist.

Vorzugsweise werden die erste siliciumorganische Verbindung und die zweite siliciumorganische Verbindung in einem solchen molaren Verhältnis eingesetzt, dass auf jede Carbamat-Einheit der Formel G-I der ersten siliciumorganischen Verbindung zumindest ein Molekül der zweiten siliciumorganischen Verbindung kommt. Es kann vorgesehen sein, dass das molare Verhältnis der ersten siliciumorganischen Verbindung zu der zweiten siliciumorganischen Verbindung 1 : n x (1 bis 1,5) beträgt, besonders bevorzugt 1 : n x (1 bis 1,2), wobei n die Zahl der Carbamat-Einheiten der Formel G-I ist. Soll ein Monoisocyanat hergestellt werden, so weist die erste siliciumorganische Verbindung eine Carbamat-Einheit der Formel G-I auf, so dass n gleich 1 ist. Soll ein Diisocyanat hergestellt werden, so weist die erste siliciumorganische Verbindung zwei Carbamat-Einheiten der Formel G-I auf, so dass n gleich 2 ist. Soll ein Triisocyanat hergestellt werden, so weist die erste siliciumorganische Verbindung drei Carbamat-Einheiten der Formel G-I auf, so dass n gleich 3 ist. Ein leichter Überschuss der zweiten siliciumorganischen Verbindung ist, bezogen auf die Zahl der Carbamat-Einheiten der ersten siliciumorganischen Verbindung, bevorzugt. Wird eine Hilfsbase verwendet, so können die zweite siliciumorganische Verbindung und die Hilfsbase in einem molaren Verhältnis von 1 : 1 bis 1,5, bevorzugt von 1 : 1 bis 1,2 verwendet werden.

Die Silylierung wird vorzugsweise bei einer Temperatur zwischen 0 und 50 °C, stärker bevorzugt bei 0 bis 25 °C und besonders bevorzugt bei Raumtemperatur durchgeführt. Unter Raumtemperatur wird eine Temperatur zwischen 15 und 25 °C verstanden. Die Silylierung wird vorzugsweise unter Umgebungsdruck durchgeführt. Sie kann unter einer Inertgasatmosphäre durchgeführt werden, wobei als Inertgas beispielsweise Stickstoff oder Argon verwendet werden können.

Die Thermolyse ist ein Verfahren zum thermischen Cracken. Die Thermolyse wird vorzugsweise bei einer Temperatur von 100 bis 400 °C, stärker bevorzugt bei einer Temperatur von 150 bis 350 °C und besonders bevorzugt bei einer Temperatur von 230 bis 300 °C durchgeführt. Die Temperatur sollte 400 °C nicht übersteigen, um eine Reaktion von Isocyanaten untereinander unter Bildung von Di-, Tri- oder Oligomeren zu verhindern. Die Thermolyse wird vorzugsweise ohne Einsatz von Lösungsmittel durchgeführt. Die Thermolyse wird vorzugsweise unter Umgebungsdruck durchgeführt. Sie kann unter einer Inertgasatmosphäre durchgeführt werden, wobei als Inertgas beispielsweise Stickstoff oder Argon verwendet werden können.

Die Thermolyse kann unter Anwendung eines Temperaturprofils durchgeführt werden. Das Temperaturprofil kann zwei oder mehr Temperaturstufen umfassen, wobei jede Temperaturstufe für einen vorgegebenen Zeitraum angewendet wird. Jede der angewendeten Temperaturstufen kann um 20 bis 80 °C, beispielsweise um 30 bis 50 °C, über dem zuvor angewendeten Temperaturbereich liegen. Der vorgegebene Zeitraum kann zwischen 1 min und 2 h liegen.

Das erfindungsgemäße Verfahren sieht die Verwendung der ersten siliciumorganischen Verbindung vor, die eine Einheit G-I aufweist, beispielsweise eine Verbindung der allgemeinen Formel II. Diese erste siliciumorganische Verbindung kann durch Insertion von Kohlenstoffdioxid in ein Aminosilan hergestellt werden. Unter einem Aminosilan wird dabei eine Verbindung verstanden, die durch Silylierung einer primären Amingruppe unter Erhalt einer direkten N-Si-Bindung erhalten wurde. Schema 5 veranschaulicht die Herstellung einer Verbindung der allgemeinen Formel II-A durch Insertion von Kohlenstoffdioxid in ein Aminosilan der allgemeinen Formel V-A. 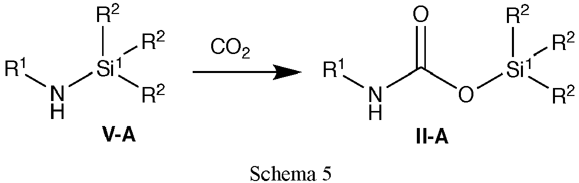 In der Verbindung der allgemeinen Formel V-A haben R¹ und R² die im Zusammenhang mit der allgemeinen Formel II angegebenen Bedeutungen. Schema 6 veranschaulicht die Herstellung einer Verbindung der allgemeinen Formel II-B durch Insertion von Kohlenstoffdioxid in ein Aminosilan der allgemeinen Formel V-B. 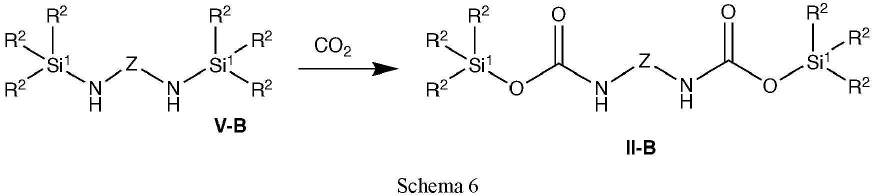 Das Aminosilan der allgemeinen Formel V-B ist eine Verbindung, die durch Silylierung von zwei endständigen, primären Amingruppen eines Moleküls unter Erhalt von zwei direkten N-Si-Bindung erhalten wurde. Sie kann als zweifach monosilyliertes primäres Diamin angesehen werden. In der Verbindung der allgemeinen Formel V-B haben R² und Z die im Zusammenhang mit der allgemeinen Formel II angegebenen Bedeutungen.

Die Insertion von Kohlenstoffdioxid kann durch Einleiten von gasförmigen Kohlenstoffdioxid in das Aminosilan oder eine Lösung des Aminosilans in einem Lösungsmittel bewirkt werden. Bei dem Lösungsmittel handelt es sich vorzugsweise um ein aprotisches organisches Lösungsmittel, beispielsweise um Tetrahydrofuran, Diethylether, Toluol, n-Pentan oder n-Hexan, wobei Tetrahydrofuran besonders bevorzugt ist. Die Insertion kann unter Umgebungsdruck oder unter Überdruck durchgeführt werden. Die Anwendung eines Überdrucks ist insbesondere dann zweckmäßig, wenn R¹ eine substituierte oder unsubstituierte aromatische Gruppe ist oder wenn Z eine substituierte oder unsubstituierte aromatische Gruppe ist. Vorzugsweise beträgt der Überdruck zwischen 1,5 und 10 bar. Die Insertion kann unter einer Inertgasatmosphäre durchgeführt werden, wobei als Inertgas beispielsweise Stickstoff oder Argon verwendet werden können. Weitere Einzelheiten zur Insertion von Kohlenstoffdioxid in ein Aminosilan können beispielsweise DE 10 2009 045 849 A1 entnommen werden.

Aminosilane, insbesondere die Aminosilane der allgemeinen Formel V-A und V-B, können aus substituierten oder unsubstituierten, aliphatischen oder aromatischen primären Aminen durch Silylierung der Amingruppe mit einem Silylierungsmittel hergestellt werden. Als Silylierungsmittel können die zweite siliciumorganische Verbindung, beispielsweise die Verbindung der allgemeinen Formel III, oder andere Silylierungsmittel eingesetzt werden. Schema 7 zeigt die Herstellung eines Aminosilans der allgemeinen Formel V-A durch Silylierung eines primären Amins der allgemeinen Formel VI-A. 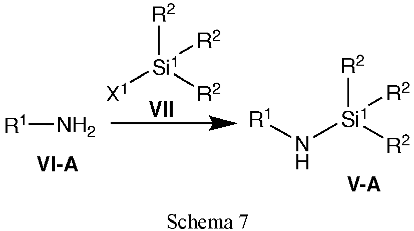 In der Verbindung der allgemeinen Formel VI-A hat R¹ die im Zusammenhang mit der allgemeinen Formel II angegebenen Bedeutungen. Bei der Verbindung der allgemeinen Formel VII handelt es sich um ein Beispiel eines Silylierungsmittels. In der Verbindung der allgemeinen Formel VII
ist X¹ aus der Gruppe ausgewählt, die aus einem Halogen, -CN, -OCN, -SCN, -N₃, einem Sulfonat und einer Gruppe -O-R⁴ besteht;
hat R² bei jedem Auftreten die im Zusammenhang mit der Verbindung der allgemeinen Formel II angegebenen Bedeutungen;
haben R⁴ bei jedem Auftreten und R⁵ die im Zusammenhang mit Formel III angegebenen Bedeutungen. Es können auch andere Silylierungsmittel eingesetzt werden. Beispiele anderer Silylierungsmittel umfassen, ohne darauf beschränkt zu sein, Aminosilane, die eine Transaminierung oder eine Transsilylierung ermöglichen, und Silazane. Weitere Beispiele geeigneter Silylierungsmittel sind in Tabelle 1 angegeben.

Die Verbindung der allgemeinen Formel VII ist ein Silan, das eine Abgangsgruppe X¹ trägt. Zur Silylierung des primären Amins kann ein - im Vergleich zur Silylierung mit der Verbindung der allgemeinen Formel III - milderes Silylierungsmittel gewählt werden. Ein Silylierungsmittel wird als milder angesehen, wenn es weniger reaktiv ist. Der Einsatz eines milderen Silylierungsmittels ist möglich, weil ein Wasserstoffatom einer primären Amingruppe leichter silyliert werden kann als das Wasserstoffatom einer Carbamat-Gruppe.

Vorzugsweise ist X¹ aus der Gruppe ausgewählt, die aus einem Halogen, einem Sulfonat und einer Gruppe -O-R⁴ besteht. Es kann vorgesehen sein, dass X¹ ein Sulfonat, also eine R^{S}-SO₂-O-Gruppe ist. R^{S} kann beispielsweise eine verzweigte oder unverzweigte substituierte oder unsubstituierte C₁-C₆-Alkylgruppe, eine Arylgruppe oder eine Alkylarylgruppe sein. Vorzugsweise ist R^{S} CH₃-, CF₃- oder CH₃-C₆H₄-. Das Sulfonat kann beispielsweise aus der Gruppe ausgewählt sein, die aus einer Toluolsulfonsäureester-Gruppe, einer Methylsulfonsäureester-Gruppe und einer Trifluormethylsulfonsäureester-Gruppe besteht. In der Verbindung der allgemeinen Formel VII ist X¹ vorzugsweise aus der Gruppe ausgewählt, die aus Chlor, einer Toluolsulfonsäureester-Gruppe, einer Methylsulfonsäureester-Gruppe und einer Trifluormethylsulfonsäureester-Gruppe besteht. Bevorzugt ist X¹ Chlor.

Vorzugsweise ist in der Verbindung der allgemeinen Formel VII R² bei jedem Auftreten eine unsubstituierte Alkylgruppe mit 1 bis 6 Kohlenstoffatomen und ist X¹ Chlor. In diesem Fall ist die Verbindung der allgemeinen Formel VII ein Trialkylchlorsilan. Eine besonders bevorzugte Verbindung der allgemeinen Formel VII ist Trimethylchlorsilan. Dabei handelt es sich um ein preiswertes Silylierungsmittel.

Weist das primäre Amin weitere primäre Aminogruppen auf, so werden diese Aminogruppen ebenfalls silyliert. Schema 8 zeigt die Herstellung eines Aminosilans der allgemeinen Formel V-B durch Silylierung eines primären Amins der allgemeinen Formel VI-B. Verbindung V-B ist ein Amin, das zwei primäre Aminogruppen aufweist.

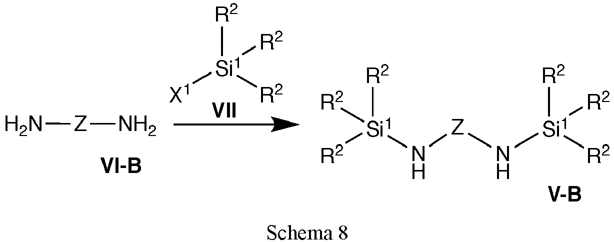

In der Verbindung der allgemeinen Formel VI-B hat Z die im Zusammenhang mit der allgemeinen Formel II angegebenen Bedeutungen. Die Verbindung der allgemeinen Formel VII ist vorstehend im Zusammenhang mit Schema 7 beschrieben worden.

Die Silylierung des primären Amins, beispielsweise des primären Amins der allgemeinen Formel VI-A oder VI-B, wird vorzugsweise in Gegenwart einer Hilfsbase durchgeführt. Die Hilfsbase dient zur Bindung der bei der Silylierung von dem Silylierungsmittel, z. B. der Verbindung der allgemeinen Formel VII, abgespaltenen Abgangsgruppe sowie des Wasserstoffatoms, das von der NH-Gruppe der Verbindung der allgemeinen Formel II abgespalten wird. Diese Gruppe ist die Gruppe X¹ in der Verbindung der allgemeinen Formel VII. Bei der Hilfsbase handelt es sich vorzugsweise um ein Amin, beispielsweise um ein Trialkylamin, wie Triethylamin, oder Anilin. Eine bevorzugte Hilfsbase ist ein Triethylamin. Es kann vorgesehen sein, dass zur Herstellung einer Verbindung der allgemeinen Formel I eine Hilfsbase eingesetzt wird, deren aliphatischer oder aromatischer Rest dem aliphatischen bzw. aromatischen Rest der Verbindung der allgemeinen Formel I entspricht. Beispielsweise kann zur Herstellung einer Verbindung der allgemeinen Formel I-A eine Verbindung der allgemeinen Formel VI-A als Hilfsbase eingesetzt werden. Zur Herstellung von Benzyl-isocyanat ist somit Benzylamin eine geeignete Hilfsbase, für die Herstellung von Phenylisocyanat Anilin. Die Verbindung der allgemeinen Formel VI-A oder VI-B ist dann nicht nur Ausgangsstoff, sondern auch Hilfsbase. Es ist deshalb zweckmäßig, die Verbindung der allgemeinen Formel VI-A oder VI-B im Überschuss zu dem Silylierungsmittel einzusetzen, beispielsweise in einem molaren Verhältnis von 2 : 1. Kommt auf 1 Molekül des Silylierungsmittels Trimethylchlorsilan 1 Molekül einer Verbindung der allgemeinen Formel VI-A oder VI-B, so bildet sich HCl, was wiederum von einem zweiten Molekül der Verbindung der allgemeinen Formel VI-A bzw. VI-B abgefangen wird. Somit verbraucht 1 Molekül Trimethylchlorsilan 2 Moleküle der Verbindung der allgemeinen Formel VI-A bzw. VI-B. Ohne den Einsatz einer Hilfsbase kann die Gefahr bestehen, dass die Abgangsgruppe eine reaktive Säure bildet, die die Bildung eines Aminosilans, z. B. der Verbindung der allgemeinen Formel V-A oder V-B, beeinträchtigt. Wird ein Trialkylchorsilan als Silylierungsmittel eingesetzt, entsteht bei der Silylierung Chlorwasserstoff, der mit der Hilfsbase abgefangen wird. Handelt es sich bei dem Silylierungsmittel um Trimethylchlorsilan und bei der Hilfsbase um Triethylamin, wird bei der Silylierung Triethylaminhydrochlorid ((CH₃CH₂)₃N*HCl) gebildet.

Die Silylierung des primären Amins, beispielsweise des primären Amins der allgemeinen Formel VI-A oder VI-B, kann unter den Bedingungen vorgenommen werden, die im Zusammenhang mit der Silylierung der ersten organischen Verbindung, beispielsweise der Verbindung der allgemeinen Formel II vorstehend beschrieben sind. Vorzugsweise wird die Silylierung des primären Amins bei Raumtemperatur und Umgebungsdruck durchgeführt. Ein Katalysator ist nicht erforderlich. Die Aminosilane werden in hohen Ausbeuten erhalten.

Das erfindungsgemäße Verfahren kann ein zweistufiges Verfahren sein. In diesem Fall geht die Herstellung einer Isocyanat-Verbindung von der ersten siliciumorganischen Verbindung (z. B. einer Verbindung der allgemeinen Formel II) aus. Das zweistufige Verfahren sieht die Silylierung der ersten siliciumorganischen Verbindung unter Erhalt der Verbindung der dritten siliciumorganischen Verbindung (im Fall einer Verbindung der allgemeinen Formel II ist das eine Verbindung der allgemeinen Formel IV) und die anschließende Thermolyse der dritten siliciumorganischen Verbindung zu der Isocyanat-Verbindung (im Fall einer Verbindung der allgemeinen Formel IV ist das eine Verbindung der allgemeinen Formel I) vor. Das zweistufige Verfahren kann bei Umgebungsdruck durchgeführt werden. Unter Umgebungsdruck kann atmosphärischer Druck verstanden werden. Das zweistufige Verfahren kann unter einer Inertgasatmosphäre durchgeführt werden.

Geht die Herstellung einer Isocyanat-Verbindung hingegen von einem primären Amin aus, so ist das erfindungsgemäße Verfahren ein vierstufiges Verfahren. Das vierstufige Verfahren sieht die Silylierung eines primären Amins unter Erhalt eines Aminosilans (1. Stufe), die Insertion von Kohlenstoffdioxid in das Aminosilan unter Erhalt der ersten siliciumorganischen Verbindung (z. B. einer Verbindung der allgemeinen Formel II) (2. Stufe), die Silylierung der ersten siliciumorganischen Verbindung unter Erhalt der Verbindung der dritten siliciumorganischen Verbindung (im Fall einer Verbindung der allgemeinen Formel II ist das eine Verbindung der allgemeinen Formel IV) (3. Stufe) und die anschließende Thermolyse der dritten siliciumorganischen Verbindung zu der Isocyanat-Verbindung (im Fall einer Verbindung der allgemeinen Formel IV ist das eine Verbindung der allgemeinen Formel I) (4. Stufe) vor. Schema 9 veranschaulicht die Herstellung einer Isocyanat-Verbindung mittels des vierstufigen Verfahrens.

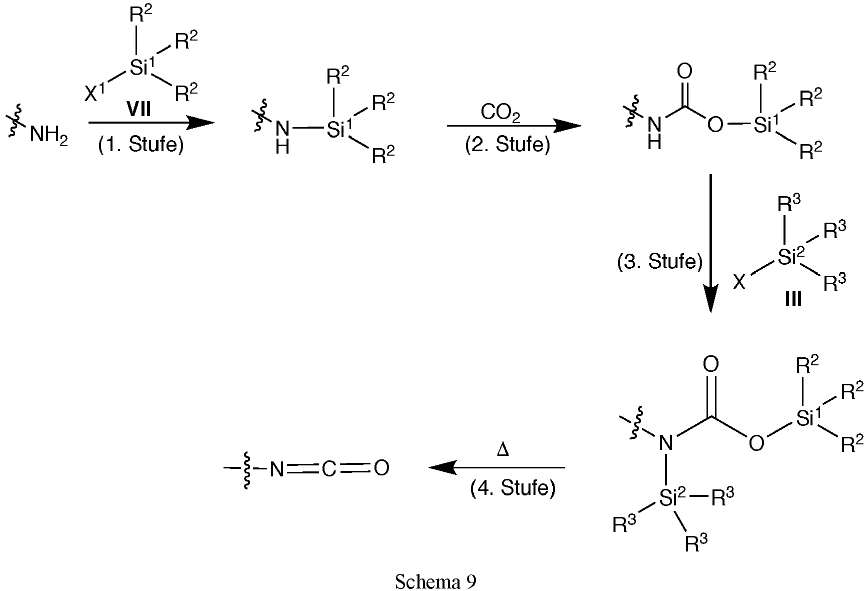

Die vierstufige Synthese von Isocyanaten, beispielsweise von Mono- und Diisocyanaten, umfasst die Synthese von Aminosilanen aus primären Aminen und einem Silylierungsmittel wie z. B. Trialkylchlorsilan (Stufe 1), die Umsetzung der Aminosilane mit CO₂ zu *O*-silylierten Carbamaten (Stufe 2), gefolgt von einem zweiten Silylierungschritt (Stufe 3) unter Erhalt von *N*,*O*-bissilylierten Carbamaten und der finalen Thermolyse der bissilylierten CO₂-Insertionsprodukte, d. h. der *N*,*O*-bissilylierten Carbamate, (Stufe 4). Das vierstufige Verfahren verzichtet auf Phosgen, setzt auf CO₂ als Kohlen- und Sauerstoffquelle und läuft ohne Katalysatoren bei moderaten Temperaturen ab. Auch die beiden ersten Stufen (Stufen 1 und 2) zeichnen sich durch die Vorteile des gesamten erfindungsgemäßen Verfahrens, wie u. a. gute Ausbeuten, geringe Reaktionswärmen etc. aus. Alle vier Stufen können unter Umgebungsdruck durchgeführt werden. Unter Umgebungsdruck kann atmosphärischer Druck verstanden werden. Das zweistufige Verfahren kann unter einer Inertgasatmosphäre durchgeführt werden. Der Einsatz von superkritischem CO₂ ist nicht vorgesehen.

Es ist ein besonderer Vorteil des erfindungsgemäßen Verfahrens, dass bei der Thermolyse der dritten siliciumorganischen Verbindung, beispielsweise einer Verbindung der allgemeinen Formel IV, neben der Zielverbindung, d. h. einer Isocyanat-Verbindung, als Koppelprodukt Siloxane gebildet werden. Bei den Siloxanen handelt es sich im einfachsten Fall um Disiloxane. Schema 10 zeigt die Thermolyse einer Verbindung der allgemeinen Formel IV, wobei neben der Verbindung der allgemeinen Formel I ein Disiloxan der allgemeinen Formel VIII gebildet wird: 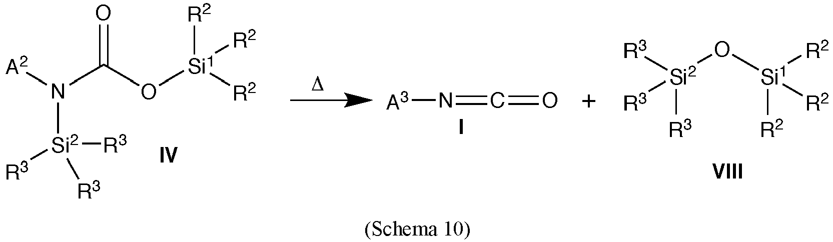 In der Verbindung der allgemeinen Formel VIII haben R² bei jedem Auftreten die im Zusammenhang mit der Verbindung der allgemeinen Formel II angegebenen Bedeutungen und R³ bei jedem Auftreten die im Zusammenhang mit der Verbindung der allgemeinen Formel III angegebenen Bedeutungen. Sind alle R² und alle R³ jeweils Methyl, so ist die Verbindung der allgemeinen Formel VIII Hexamethyldisiloxan. Hexamethyldisiloxan hat verschiedene Verwendungen, z. B. kann es mit Halogenen oder Halogenwasserstoffsäure in Trimethylsilylhalogenide umgewandelt werden. Es findet weiterhin bei der Herstellung von Silikonölen Anwendung zur Einstellung der Molmassenverteilungen.

Der Ausdruck "aliphatisch" bezieht sich, sofern nichts anderes angegeben ist, auf eine verzweigte oder unverzweigte oder cyclische Kohlenwasserstoffkomponente, einschließlich einer Komponente, die sowohl cyclische als auch Kettenelemente enthält, die vollständig gesättigt oder einfach oder mehrfach ungesättigt sein können. Beispiele für gesättigte Kohlenwasserstoffkomponenten umfassen Alkylgruppen, Alkylengruppen und Cycloalkylgruppen. Beispiele für ungesättigte Kohlenwasserstoffkomponenten umfassen Alkenylgruppen und Alkinylgruppen. Die aliphatische Kohlenwasserstoffkomponente kann gegebenenfalls ein oder mehrere, beispielsweise ein bis vier Heteroatome enthalten, die aus der Gruppe ausgewählt sind, die aus O, N, S, Si und Ge besteht.

Der Ausdruck "aromatisch" bezieht sich, sofern nichts anderes angegeben ist, auf eine Kohlenwasserstoffkomponente, die zumindest einen aromatischen Ring aufweist, einschließlich einer Komponente, die sowohl zumindest einen aromatischen als auch zumindest ein Kettenelement enthält, das vollständig gesättigt oder einfach oder mehrfach ungesättigt sein kann. Beispiele für aromatische Ringe umfassen Arylgruppen, Heteroarylgruppen, Arylengruppen und Heteroarylengruppen. Beispiele für Kettenelemente umfassen Alkylengruppe. Beispiele für aromatische Gruppen, die zumindest einen aromatischen Ring als auch zumindest ein Kettenelement enthalten sind, umfassen Alkylenarylengruppen, Arylenbis(alkylen)gruppen und Alkylenbis(arylen)gruppen. Die aromatische Kohlenwasserstoffkomponente kann gegebenenfalls ein oder mehrere, beispielsweise ein bis vier Heteroatome enthalten, die aus der Gruppe ausgewählt sind, die aus O, N, S, Si und Ge besteht.

Der Ausdruck "Alkyl" bezieht sich, sofern nichts anderes angegeben ist, insbesondere auf eine gesättigte aliphatische Kohlenwasserstoff-Gruppe mit einer verzweigten oder unverzweigten Kohlenstoffkette mit 1 bis 12 Kohlenstoffatomen, vorzugsweise 1 bis 8 Kohlenstoffatomen und besonders bevorzugt 1 bis 6 Kohlenstoffatomen. Beispiele von Alkylgruppen umfassen, sind aber nicht beschränkt auf Methyl, Ethyl, Propyl, Isopropyl, Isobutyl, *sec*-Butyl, *tert*-Butyl, Pentyl, *n*-Hexyl, Octyl, Dodecyl und dergleichen. Die Alkylgruppe kann gegebenenfalls mit einem oder mehreren Substituenten substituiert sein, wobei jeder Substituent unabhängig Alkyl, Alkoxy, Halogen, Halogenalkyl, Amino, Monoalkylamino, Dialkylamino, SiR^{z}₃, Si(OR^{z})₃ , SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Die Alkylgruppe kann teil- oder perfluoriert sein. Die Alkylgruppe kann gegebenenfalls ein oder mehrere Heteroatome enthalten, die aus der Gruppe ausgewählt sind, die aus O, N, S, Si und Ge besteht. Die Alkylgruppe kann eine oder mehrere Ketten, die aus zwei oder mehr, beispielsweise 2 bis 12 Si-Atomen bestehen, enthalten.

Der Ausdruck "Heteroalkyl" bezieht sich, sofern nichts anderes angegeben ist, auf eine Alkylgruppe, wie hierin definiert, wobei ein, zwei oder drei Wasserstoffatome durch einen Substituenten, unabhängig ausgewählt aus der Gruppe, bestehend aus -OR^{a}, -NR^{b}R^{c}, -S(O)_{w}R^{d} (worin w eine Ganzzahl von 0 bis 2 ist), SiR^{z}₃, Si(OR^{z})₃ und SiR^{z}(OR^{z})₂, ersetzt wurden, unter der Voraussetzung, dass der Anlagerungspunkt des Heteroalkylrestes ein Kohlenstoffatom ist, wobei R^{a} Acyl, Alkyl, Cycloalkyl oder Cycloalkylalkyl ist; R^{b} und R^{c} unabhängig voneinander Acyl, Alkyl, Cycloalkyl oder Cycloalkylalkyl sind; und wenn w 0 ist, R^{d} Alkyl, Cycloalkyl oder Cycloalkylalkyl ist, und wenn w 1 oder 2 ist, R^{d} Alkyl, Cycloalkyl oder Cycloalkylalkyl ist; R^{z} bei jedem Auftreten unabhängig voneinander Wasserstoff oder Alkyl, wie hierin definiert, ausgenommen SiH₃ ist.

Der Ausdruck "Cycloalkyl" bezieht sich, sofern nichts anderes angegeben ist, insbesondere auf gesättigte, carbocyclische Gruppen, die aus mono- oder bicyclischen Ringen bestehen und 3 bis 12 Ringatome aufweisen. Die Cycloalkylgruppe kann gegebenenfalls mit einem oder mehreren Substituenten substituiert sein, wobei jeder Substituent unabhängig Alkyl, Alkoxy, Amino, Monoalkylamino, Dialkylamino, SiR^{z}₃, Si(OR^{z})₃, SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Beispiele von Cycloalkylgruppen umfassen, sind aber nicht beschränkt auf, Cyclopropyl, Cyclobutyl, Cyclopentyl, Cyclohexyl, Cycloheptyl und dergleichen. Die Cycloalkylgruppe kann teil- oder perfluoriert sein. Die Cycloalkylgruppe kann gegebenenfalls ein oder mehrere Heteroatome enthalten, die aus der Gruppe ausgewählt sind, die aus O, N, S, Si und Ge besteht.

Der Ausdruck "Heterocycloalkyl" bezieht sich, sofern nichts anderes angegeben ist, auf einen gesättigten cyclischen Ring mit 5 bis 12 Ringatomen, wobei 1 bis 4 der Ringatome Heteroatome, die aus einem oder mehreren von N, O und S ausgewählt sind, und die verbliebenen Ringatome Kohlenstoffatome sind. Beispiele von Heterocycloalkylgruppen umfassen, sind jedoch nicht beschränkt auf Piperidinyl, Piperazinyl, Homopiperazinyl, Azepinyl, Pyrrolidinyl, Pyrazolidinyl, Imidazolinyl, Imidazolidinyl, Oxazolidinyl, Isoxazolidinyl, Morpholinyl, Thiazolidinyl, Isothiazolidinyl, Chinuclidinyl, Tetrahydrofuryl, Tetrahydropyranyl, Thiomorpholinyl, Dihydrochinolinyl und 1,4-Diazepan. Die Heterocycloalkylgruppe kann gegebenenfalls mit einem oder mehreren Substituenten substituiert sein, wobei jeder Substituent unabhängig Alkyl, Alkoxy, Amino, Monoalkylamino, Dialkylamino, SiR^{z}₃, Si(OR^{z})₃, SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Die Heterocycloalkylgruppe kann teil- oder perfluoriert sein.

Der Ausdruck "Alkenyl" bezieht sich, sofern nichts anderes angegeben ist, insbesondere auf eine ungesättigte aliphatische Kohlenwasserstoff-Gruppe mit einer verzweigten oder unverzweigten Kohlenstoffkette mit 2 bis 12 Kohlenstoffatomen, vorzugsweise 2 bis 8 Kohlenstoffatomen und besonders bevorzugt 2 bis 6 Kohlenstoffatomen, die mindestens eine olefinische Doppelbindung und stärker bevorzugt eine einzige Doppelbindung aufweist. Beispiele von Alkenylgruppen umfassen, sind aber nicht beschränkt auf Vinyl, Allyl, Methallyl, 1,1-Dimethylallyl, Propenyl, Butenyl, Pentadienyl, Hexenyl, Octenyl und dergleichen. Eine Allylgruppe ist bevorzugt. Die Alkenylgruppe kann gegebenenfalls mit einem oder mehreren Substituenten substituiert sein, wobei jeder Substituent unabhängig Alkyl, Alkoxy, Halogen, Halogenalkyl, Amino, Monoalkylamino, Dialkylamino, SiR^{z}₃, Si(OR^{z})₃, SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Die Alkenylgruppe kann teil- oder perfluoriert sein. Die Alkenylgruppe kann gegebenenfalls ein oder mehrere Heteroatome enthalten, die aus der Gruppe ausgewählt sind, die aus O, N, S, Si und Ge besteht. Die Alkenylgruppe kann eine oder mehrere Ketten, die aus zwei oder mehr, beispielsweise 2 bis 12 Si-Atomen bestehen, enthalten.

Der Ausdruck "Alkinyl" bezieht sich, sofern nichts anderes angegeben ist, insbesondere auf eine ungesättigte aliphatische Kohlenwasserstoff-Gruppe mit einer verzweigten oder unverzweigten Kohlenstoffkette mit 2 bis 12 Kohlenstoffatomen, vorzugsweise 2 bis 8 Kohlenstoffatomen und besonders bevorzugt 2 bis 6 Kohlenstoffatomen, die mindestens eine olefinische Dreifachbindung und stärker bevorzugt eine einzige Dreifachbindung aufweist. Beispiele von Alkinylgruppen umfassen, sind aber nicht beschränkt auf Acetylenyl, Propargyl, n-But-2-in-1-yl und dergleichen. Eine Propargylgruppe ist bevorzugt. Die Alkinylgruppe kann gegebenenfalls mit einem oder mehreren Substituenten substituiert sein, wobei jeder Substituent unabhängig Alkyl, Alkoxy, Halogen, Halogenalkyl, Amino, Monoalkylamino, Dialkylamino SiR^{z}₃, Si(OR^{z})₃ , SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Die Alkinylgruppe kann gegebenenfalls ein oder mehrere Heteroatome enthalten, die aus der Gruppe ausgewählt sind, die aus O, N, S, Si und Ge besteht. Die Alkinylgruppe kann eine oder mehrere Ketten, die aus zwei oder mehr, beispielsweise 2 bis 12 Si-Atomen bestehen, enthalten.

Der Ausdruck "Alkoxy" bezieht sich, sofern nichts anderes angegeben ist, insbesondere auf eine Gruppe der Formel -OR, worin R eine Alkylgruppe ist, wie hierin definiert. Beispiele von Alkoxykomponenten umfassen, sind aber nicht beschränkt auf Methoxy, Ethoxy, Isopropoxy und dergleichen. Die Alkoxygruppe kann gegebenenfalls mit einem oder mehreren Substituenten substituiert sein, wobei jeder Substituent unabhängig Alkyl, Alkoxy, Halogen, Halogenalkyl, Amino, Monoalkylamino, Dialkylamino, SiR^{z}₃, Si(OR^{z})₃, SiR ^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben.

Der Ausdruck "Acyl" bezieht sich auf eine Gruppe der Formel -C(=O)R, wobei R Alkyl, wie hierin definiert, ist.

Der Ausdruck "Halogen" bezieht sich auf Fluor, Chlor, Brom oder Iod.

Der Ausdruck "Aryl" bezieht sich, sofern nichts anderes angegeben ist, auf eine cyclische, aromatische Kohlenwasserstoffgruppe, die aus einem mono- oder bicyclischen aromatischen Ringsystem mit 5 bis 10 Ringatomen, bevorzugt 5 oder 6 Ringatomen, besteht oder ein solches Ringsystem aufweist. Die Arylgruppe kann gegebenenfalls eine substituierte Arylgruppe sein. Beispiele von Arylgruppen umfassen, sind aber nicht beschränkt auf Phenyl, Naphthyl, Naphthalenyl, Fluorenyl, Indenyl, Pentalenyl, Azulenyl, Oxydiphenyl, Biphenyl, Methylendiphenyl, Aminodiphenyl, Diphenylsulfidyl, Diphenylsulfonyl, Diphenylisopropylidenyl, Benzodioxanyl, Benzofuranyl, Benzodioxylyl, Benzopyranyl, Benzoxazinyl, Benzoxazinonyl, Benzopiperadinyl, Benzopiperazinyl, Benzopyrrolidinyl, Benzomorpholinyl, Methylendioxyphenyl, Ethylendioxyphenyl und dergleichen, einschließlich teilweise hydrierte Derivate davon. Der Ausdruck "substituierte Arylgruppe" bezieht sich insbesondere auf eine Arylgruppe, die gegebenenfalls unabhängig mit ein bis vier Substituenten, bevorzugt einem oder zwei Substituenten, ausgewählt aus Alkyl, Cycloalkyl, Heteroalkyl, Halogen, Alkoxy, Amino, Acylamino, Monoalkylamino, Dialkylamino, SiR^{z}₃, Si(OR^{z})₃ , SiR^{z}(OR^{z})₂ (worin R^{z} bei jedem Auftreten unabhängig voneinander Wasserstoff oder Alkyl, wie hierin definiert, ausgenommen SiH₃ ist), einer Siloxangruppe, Halogenalkyl, Halogenalkoxy, Alkansulfonyl, -COR (worin R Alkyl, Phenyl oder Phenylalkyl ist), -(CR'R")ₙ-COOR (worin n eine Ganzzahl von 0 bis 5 ist, R' und R" unabhängig voneinander Wasserstoff oder Alkyl sind, und R Alkyl, Cycloalkyl, Cycloalkylalkyl, Phenyl oder Phenylalkyl ist) oder -(CR'R")ₙ-CONR^{a'}R^{b'} (worin n eine Ganzzahl von 0 bis 5 ist, R' und R" unabhängig voneinander Wasserstoff oder Alkyl sind und R^{a'} und R^{b'} unabhängig voneinander Alkyl, Cycloalkyl, Cycloalkylalkyl, Phenyl oder Phenylalkyl sind), substituiert ist.

Der Ausdruck "Heteroaryl" bezieht sich, sofern nichts anderes angegeben ist, auf eine monocyclische oder bicyclische Gruppe mit 5 bis 10, stärker bevorzugt 5 bis 6 Ringatomen mit mindestens einem aromatischen Ring und ferner enthaltend ein, zwei, drei oder vier Ringheteroatome, ausgewählt aus N, O und S, wobei die verbleibenden Ringatome C sind. Das Heteroaryl kann gegebenenfalls mit einem, zwei, drei oder vier Substituenten substituiert sein, wobei jeder Substituent unabhängig Amino ist, oder für den nicht aromatischen Teil des cyclischen Rings auch durch Oxo, sofern nicht speziell anders angegeben. Beispiele von Heteroarylgruppen umfassen, sind jedoch nicht beschränkt auf gegebenenfalls substituiertes Imidazolyl, gegebenenfalls substituiertes Oxazolyl, gegebenenfalls substituiertes Thiazolyl, gegebenenfalls substituiertes Pyrazinyl, gegebenenfalls substituiertes Pyrrolyl, gegebenenfalls substituiertes Pyridinyl, gegebenenfalls substituiertes Pyrimidinyl, gegebenenfalls substituiertes Indonyl, gegebenenfalls substituiertes Isochinolinyl, gegebenenfalls substituiertes Carbazol-9-yl, gegebenenfalls substituiertes Furanyl, gegebenenfalls substituiertes Benzofuranyl, gegebenenfalls substituiertes Benzo[1,2,3]thiadiazolyl, gegebenenfalls substituiertes Benzo[b]thiophenyl, gegebenenfalls substituiertes 9H-Thioxanthenyl und gegebenenfalls substituiertes Thieno[2,3-c]pyridinyl.

Der Ausdruck "Alkylaryl" bezieht sich, sofern nichts anderes angegeben ist, insbesondere auf eine Gruppe der Formel -R^{g}R^{h}, wobei R^{g} eine Arylengruppe ist und R^{h} eine Alkylgruppe ist, wie hierin definiert. Die Alkylarylgruppe kann gegebenenfalls eine substituierte Alkylarylgruppe sein. Beispiele von Alkylarylgruppen umfassen, sind aber nicht beschränkt auf o-Tolyl, m-Tolyl p-Tolyl, o*-tert-*Butylphenyl, m*-tert-* Butylphenyl, p-tert-Butylphenyl und dergleichen.

Der Ausdruck "Arylalkyl" bezieht sich, sofern nichts anderes angegeben ist, insbesondere auf eine Gruppe der Formel -R^{e}R^{f}, wobei R^{e} eine Alkylengruppe ist und R^{f} eine Arylgruppe ist, wie hierin definiert. Die Arylalkylgruppe kann gegebenenfalls eine substituierte Arylalkylgruppe sein. Beispiele von Arylalkylgruppen umfassen, sind aber nicht beschränkt auf Benzyl, Phenylethyl, 3-(3-Chlorphenyl)-2-methylpentyl und dergleichen.

Der Ausdruck "Alkylen" bezieht sich dabei insbesondere auf eine zweiwertige gesättigte aliphatische Kohlenwasserstoff-Gruppe mit einer verzweigten oder unverzweigten Kohlenstoffkette mit 1 bis 12 Kohlenstoffatomen, vorzugsweise 1 bis 8 Kohlenstoffatomen und besonders bevorzugt 1 bis 6 Kohlenstoffatomen. Beispiele von Alkylengruppen umfassen, sind aber nicht beschränkt auf Methylen, Ethylen, Propylen, Butylen und dergleichen. Die Alkylengruppe kann gegebenenfalls mit einem oder mehreren Substituenten substituiert sein, wobei jeder Substituent unabhängig Alkyl, Alkoxy, Halogen, Halogenalkyl, Amino, Monoalkylamino, Dialkylamino, SiR^{z}₃, Si(OR^{z})₃, SiR^{z}(OR^{z})₂, worin R^{z} bei jedem Auftreten unabhängig voneinander Wasserstoff oder Alkyl, wie hierin definiert, ausgenommen SiH₃ ist, oder eine Siloxangruppe ist, wenn nicht speziell anders angegeben. Die Alkylengruppe kann teil- oder perfluoriert sein. Die Alkylengruppe kann gegebenenfalls ein oder mehrere Heteroatome enthalten, die aus der Gruppe ausgewählt sind, die aus O, N, S, Si und Ge besteht. Die Alkylengruppe kann eine oder mehrere Ketten, die aus zwei oder mehr, beispielsweise 2 bis 12 Si-Atomen bestehen, enthalten.

Der Ausdruck "Arylen" bezieht sich, sofern nichts anderes angegeben ist, auf eine zweiwertige cyclische, aromatische Kohlenwasserstoffgruppe, die aus einem mono- oder bicyclischen aromatischen Ringsystem mit 5 bis 10 Ringatomen, bevorzugt 5 oder 6 Ringatomen, besteht. Die Arylengruppe kann gegebenenfalls eine substituierte Arylengruppe sein. Beispiele von Arylengruppen umfassen, sind aber nicht beschränkt auf Phenylen, Naphthylen. Der Ausdruck "substituierte Arylengruppe" bezieht sich insbesondere auf eine Arylengruppe, die gegebenenfalls unabhängig mit ein bis vier Substituenten, bevorzugt einem oder zwei Substituenten, ausgewählt aus Alkyl, Cycloalkyl, Heteroalkyl, Halogen, Alkoxy, Amino, Acylamino, Monoalkylamino, Dialkylamino, SiR^{z}₃, Si(OR^{z})₃, SiR^{z}(OR^{z})₂ (worin R^{z} bei jedem Auftreten unabhängig voneinander Wasserstoff oder Alkyl, wie hierin definiert, ausgenommen SiH₃ ist), einer Siloxangruppe, Halogenalkyl, Halogenalkoxy, Alkansulfonyl, -COR (worin R Alkyl, Phenyl oder Phenylalkyl ist), -(CR'R")ₙ-COOR (worin n eine Ganzzahl von 0 bis 5 ist, R' und R" unabhängig voneinander Wasserstoff oder Alkyl sind, und R Alkyl, Cycloalkyl, Cycloalkylalkyl, Phenyl oder Phenylalkyl ist) oder -(CR'R")ₙ-CONR^{a'}R^{b'} (worin n eine Ganzzahl von 0 bis 5 ist, R' und R" unabhängig voneinander Wasserstoff oder Alkyl sind und R^{a'} und R^{b'} unabhängig voneinander Alkyl, Cycloalkyl, Cycloalkylalkyl, Phenyl oder Phenylalkyl sind), substituiert ist.

Der Ausdruck "Heteroarylen" bezieht sich, sofern nichts anderes angegeben ist, auf eine zweiwertige cyclische, aromatische Kohlenwasserstoffgruppe, die aus einem mono- oder bicyclischen aromatischen Ringsystem mit 5 bis 10 Ringatomen, bevorzugt 5 oder 6 Ringatomen, besteht, wobei das Ringsystem ein, zwei, drei oder vier Ringheteroatome, ausgewählt aus N, O und S, wobei die verbleibenden Ringatome C sind, enthält. Die Heteroarylengruppe kann gegebenenfalls eine substituierte Heteroarylengruppe sein. Beispiele von Heteroarylengruppen umfassen, sind aber nicht beschränkt auf Pyrenylen und Furylen. Der Ausdruck "substituierte Heteroarylengruppe" bezieht sich insbesondere auf eine Heteroarylengruppe, die gegebenenfalls unabhängig mit ein bis vier Substituenten, bevorzugt einem oder zwei Substituenten, ausgewählt aus Alkyl, Cycloalkyl, Heteroalkyl, Halogen, Alkoxy, Amino, Acylamino, Monoalkylamino, Dialkylamino, SiR^{z}₃, Si^{z}(OR^{z})₃, SiR^{z}(OR^{z})₂ (worin R^{z} bei jedem Auftreten unabhängig voneinander Wasserstoff oder Alkyl, wie hierin definiert, ausgenommen SiH₃ ist), einer Siloxangruppe, Halogenalkyl, Halogenalkoxy, Alkansulfonyl, -COR (worin R Alkyl, Phenyl oder Phenylalkyl ist), -(CR'R")ₙ-COOR (worin n eine Ganzzahl von 0 bis 5 ist, R' und R" unabhängig voneinander Wasserstoff oder Alkyl sind, und R Alkyl, Cycloalkyl, Cycloalkylalkyl, Phenyl oder Phenylalkyl ist) oder -(CR'R")ₐ-CONR^{a'}R^{b'} (worin n eine Ganzzahl von 0 bis 5 ist, R' und R" unabhängig voneinander Wasserstoff oder Alkyl sind und R^{a'} und R^{b'} unabhängig voneinander Alkyl, Cycloalkyl, Cycloalkylalkyl, Phenyl oder Phenylalkyl sind), substituiert ist.

Der Ausdruck "Alkylenarylen" bezieht sich, sofern nichts anderes angegeben ist, insbesondere auf eine Gruppe der Formel -R^{m}Rⁿ-, wobei R^{m} eine Arylengruppe ist und Rⁿ eine Alkylengruppe ist, wie hierin definiert. Die Alkylenarylengruppe kann gegebenenfalls eine substituierte Alkylenarylengruppe sein. Beispiele von Alkylenarylengruppen umfassen, sind aber nicht beschränkt auf Phenylenmethylen, Phenylenethylen, Phenylen-n-propylen und dergleichen.

Der Ausdruck "Arylenbis(alkylen)" bezieht sich, sofern nichts anderes angegeben ist, insbesondere auf eine Gruppe der Formel -RⁿR^{m}Rⁿ-, wobei R^{m} eine Arylengruppe ist und Rⁿ bei jedem Auftreten unabhängig voneinander jeweils ein Alkylengruppe ist, wie hierin definiert. Das Arylenbis(alkylen) kann gegebenenfalls eine substituiertes Arylenbis(alkylen) sein. Beispiele von Arylenbis(alkylen) umfassen, sind aber nicht beschränkt auf Phenylenbis(methylen), Phenylenbis(ethylen), Phenylenbis(n-propylen) und dergleichen.

Der Ausdruck "Alkylenbis(arylen)" bezieht sich, sofern nichts anderes angegeben ist, insbesondere auf eine Gruppe der Formel -R^{m}RⁿR^{m}-, wobei R^{m} bei jedem Auftreten unabhängig voneinander jeweils eine Arylengruppe ist und Rⁿ eine Alkylengruppe ist, wie hierin definiert. Das Alkylenbis(arylen) kann gegebenenfalls ein substituiertes Alkylenbis(arylen) sein. Beispiele von Alkylenbis(arylen) umfassen, sind aber nicht beschränkt auf Methylenbis(phenylen), Ethylenbis(phenylen), *n*-Propylenbis(phenylen) und dergleichen.

Nach Maßgabe der Erfindung ist ferner die Verwendung einer Verbindung der allgemeinen Formel II 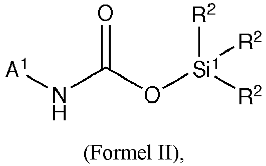 worin
A¹ eine Gruppe der allgemeinen Formel G-III ist: 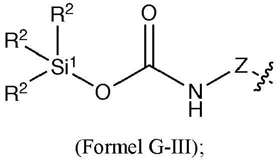
R² bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si¹ zumindest ein Rest R² gebunden ist, der nicht Wasserstoff ist;
Z eine substituierte oder unsubstituierte, aliphatische oder aromatische Gruppe ist ist; zur Herstellung einer Isocyanat-Verbindung der allgemeinen Formel I

   A³-N=C=O (Formel I),

   vorgesehen, worin
A³ eine Gruppe der Formel G-V ist: 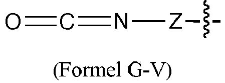
Z die in Zusammenhang mit der Verbindung der allgemeinen Formel II angegebenen Bedeutungen hat.

Nach Maßgabe der Erfindung ist außerdem die Verwendung einer Verbindung der allgemeinen Formel IV 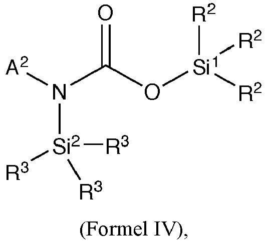 worin
A² eine Gruppe der allgemeinen Formel G-IV ist: 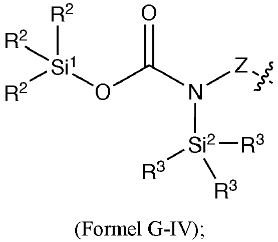
R² bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si¹ zumindest ein Rest R² gebunden ist, der nicht Wasserstoff ist;
R³ bei jedem Auftreten die in Zusammenhang mit der Verbindung der allgemeinen Formel III angegebenen Bedeutungen hat;
Z eine substituierte oder unsubstituierte, aliphatische oder aromatische Gruppe ist;

zur Herstellung einer Isocyanat-Verbindung der allgemeinen Formel I

   A³-N=C=O (Formel I),
vorgesehen, worin
   A³ eine Gruppe der Formel G-V ist: 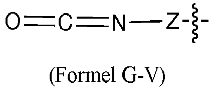
   Z die in Zusammenhang mit der Verbindung der allgemeinen Formel II angegebene Bedeutung hat.

Einzelheiten zu den erfindungsgemäßen Verwendungen können der Beschreibung des erfindungsgemäßen Verfahrens entnommen werden.

Das erfindungsgemäße Verfahren und die erfindungsgemäßen Verwendungen bieten einen sicheren, Phosgen-freien Syntheseweg für Isocyanate. Dabei werde kostengünstige Rohstoffe verwendet, Isocyanate in hohen Ausbeuten und hoher Reinheit erhalten und als Nebenprodukte nutzbare Koppelprodukte, d. h. Siloxane, erhalten. Der Syntheseweg weist einen niedrigen Energieverbrauch auf. Das ist unter anderem darauf zurückzuführen, dass der Syntheseweg mit geringeren Reaktionswärmen verbunden ist. Außerdem kommt der Syntheseweg ohne Katalysatoren aus. Das erfindungsgemäße Verfahren ermöglicht insbesondere die sichere und wirtschaftliche Synthese von Diisocyanten. Diese Diisocyante können als Monomere für die Herstellung von Polyurethanen eingesetzt werden. Das erfindungsgemäße Verfahren und die erfindungsgemäßen Verwendungen sind insbesondere für die Herstellung von aromatischen Isocyanaten geeignet, insbesondere von Isocyanaten, in denen R¹ Aryl, wie Phenyl, oder Z eine Arylengruppe enthält, wie Phenylen oder Phenylenbis(methylen), ist.

Die Erfindung wird nachstehend anhand von Ausführungsbeispielen, die die Erfindung nicht einschränken sollen, näher erläutert.

In den Beispielen wird die Herstellung der Isocyanate 11, 12 und 16 ausgehend von primären Aminen beschrieben. Die Isocyanate 11 und 12 können nach dem in Schema 11 gezeigten vierstufigen Syntheseweg hergestellt werden. 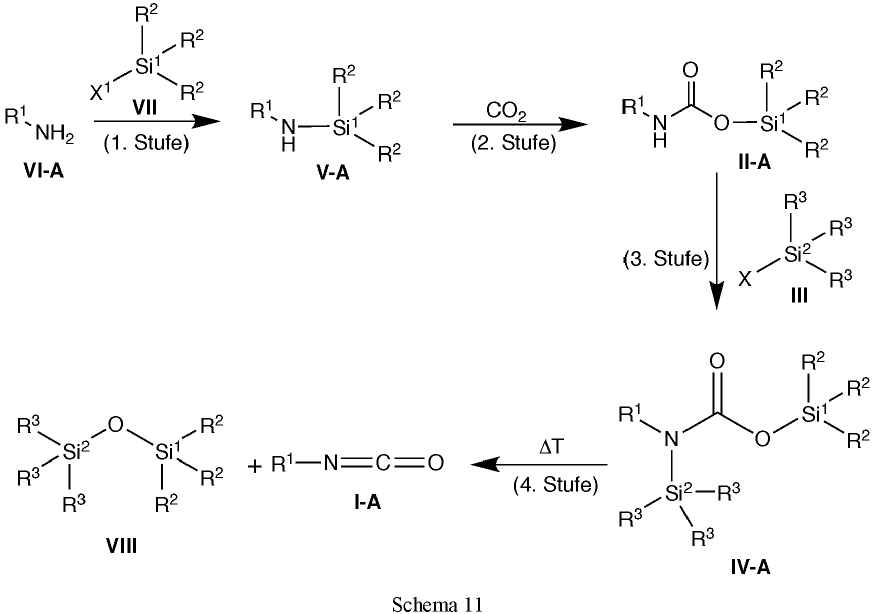 Schema 11 zeigt eine Variante des in Schema 9 gezeigten vierstufigen Syntheseweges, die zur Herstellung von Monoisocyanaten geeignet ist. Bezogen auf Schema 11 sind in Beispiel 1 R¹ Phenyl, R² und R³ bei jedem Auftreten Methyl, X¹ Chlor und X Triflat. In Beispiel 2 sind R¹ *n*-Octyl, R² und R³ bei jedem Auftreten Methyl, X¹ Chlor und X Triflat.

Mittels der in Schema 11 gezeigten Verfahrensweise kann beispielsweise auch Benzylisocyanat hergestellt werden. Bezogen auf Schema 11 ist R¹ in diesem Fall Benzyl, R² und R³ bei jedem Auftreten Methyl, X¹ Chlor und X Triflat.

Das Isocyanat 16 kann nach dem in Schema 12 gezeigten vierstufigen Syntheseweg hergestellt werden. 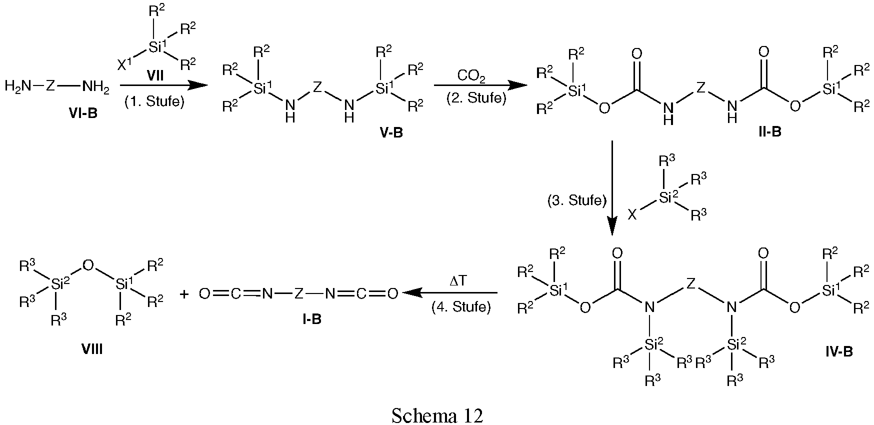 Schema 12 zeigt eine Variante des in Schema 9 gezeigten vierstufigen Syntheseweges, die zur Herstellung von Diisocyanaten geeignet ist. Bezogen auf Schema 12 sind in Beispiel 3 Z -(CH₂)₈-, R² und R³ bei jedem Auftreten Methyl, X¹ Chlor und X Triflat.

Mittels der in Schema 12 gezeigten Verfahrensweise kann beispielsweise auch 1,3-Bis(isocyanatomethyl)benzen hergestellt werden, das auch als m-Xylylendiisocynat bezeichnet wird. Bezogen auf Schema 12 ist Z in diesem Fall 1,3-Phenylenbis(methylen), R² und R³ bei jedem Auftreten Methyl, X¹ Chlor und X Triflat.

### Beispiel 1

### Synthese von Phenylisocyanat (11)

Phenylisocyanat (Verbindung **11**) wurde gemäß den in Schema 11 gezeigten Verfahren hergestellt. Phenylisocyanat ist ein Beispiel eines aromatischen Monoisocyanats.

### Stufe 1: Aminosilansvnthese

4,28 g (46,00 mmol) Anilin wurden zusammen mit 5,10 g (50,50 mmol) Triethylamin in 100 ml n-Pentan vorgelegt. Unter Rühren im Eisbad erfolgte die Zugabe von 5,12 g (47,16 mmol) Trimethylchlorsilan über einen Tropftrichter. Beim Zutropfen des Silans konnte die Bildung eines weißen Feststoffes sowie eine weiße Rauchentwicklung beobachtet werden. Nach vollständiger Zugabe des Chlorsilans wurde das Gemisch auf Raumtemperatur erwärmt und für 24 Stunden gerührt. Nach Entfernen des weißen Feststoffes durch Filtration sowie Entfernen des Lösungsmittels unter vermindertem Druck konnten 7,13 g (43,14 mmol) N Trimethylsilylanilin als klare, farblose Flüssigkeit erhalten werden. Die Ausbeute beträgt 94 %. Die Reinheit des Produktes wurde mittels NMR-Spektroskopie bestätigt.

¹H-NMR (400 MHz, C₆D₆, δ [ppm]): 7,08-6,43 (m, 5H, ArH), 3,46 (s, 1H, NH), 0,15 (s, 9H, SiMe3). ¹³C-NMR (101 MHz, C₆D₆, δ [ppm]): 148,1 (ArC), 129,6 (ArCH), 117,9 (ArCH), 116,7 (ArCH), 0,2 (SiMe3). ²⁹Si-NMR (79 MHz, C₆D₆, δ [ppm]): 2,1.

### Stufe 2: CO₂-Insertion

7,56 g (45,7 mmol) N Trimethylsilylanilin wurden mit 20 ml trockenem Tetrahydrofuran (THF) vermengt und unter inerten Bedingungen in einen großen Autoklav gefüllt. Der Autoklav wurde mit 8 bar CO₂-Überdruck beaufschlagt und für 24 Stunden unter diesen Bedingungen stehen gelassen. Der Druck wurde in regelmäßigen Abständen kontrolliert. Nach Ablauf der Reaktionszeit wurde das klare, hellgelbe Reaktionsgemisch inert in ein Schlenk-Gefäß überführt. Nach wenigen Minuten konnte die Bildung von farblosen Kristallnadeln beobachtet werden. Das Lösungsmittel wurde unter vermindertem Druck entfernt und der erhaltene, weiße Feststoff für 3 Stunden im Vakuum getrocknet. Auf diesem Weg konnten 8,62 g (41,17 mmol) N-Phenyl-O-trimethylsilyl-carbamat in einer Ausbeute von 90 % erhalten werden. Die Identität und Reinheit der Zielverbindung wurden mittels NMR-Spektroskopie bestätigt.

¹H-NMR (400 MHz, THF-d8, δ [ppm]): 8,64 (s, 1H, NH), 7,46-6,92 (m, 5H, ArH), 0,30 (s, 9H, SiMe3). ¹³C-NMR (101 MHz, THF-d8, δ [ppm]): 153,0 (CO), 140,8 (ArC), 129,4 (ArCH), 123,1 (ArCH), 118,9 (ArCH), 0,1 (SiMe3). ²⁹Si-NMR (79 MHz, THF-d8, δ [ppm]): 22,2. Elementaranalyse: berechnet N 6,69 %, C 57,38 %, H 7,22 %; gemessen N 6,79 %, C 57,14 %, H 7,053 %.

### Stufe 3: Silylierung

6,87 g (32,82 mmol) *N*-Phenyl-*O*-trimethylsilyl-carbamat wurden zusammen mit 3,40 g (33,60 mmol) Triethylamin in 100 ml *n*-Pentan gelöst. Das Gemisch wurde im Eisbad gerührt. Über einen Tropftrichter wurden 7,28 g (32,76 mmol) Trimethylsilyltriflat zugetropft. Es konnte die Bildung einer flockenartigen zweiten Phase beobachtet werden. Das Gemisch wurde anschließend auf Raumtemperatur erwärmt und für 2 Stunden bei dieser Temperatur gerührt. Die entstandene zweite Phase wurde in einem Kältegemisch aus Trockeneis und Isopropanol zum Erstarren gebracht und die überstehende Lösung abdekantiert. Das Lösungsmittel wurde unter reduziertem Druck entfernt, wobei das Ausfallen eines weißen Feststoffes beobachtet werden konnte. Der Feststoff wurde für 2 Stunden im Vakuum getrocknet. Auf diese Weise konnten mit einer Ausbeute von 83 % 7,69 g (27,32 mmol) *N*,*O-*Bis(trimethylsilyl)-*N*-phenyl-carbamat (**1**) erhalten werden. Die Identität und Reinheit des Zielproduktes wurden mittels NMR-Spektroskopie sowie Einkristall-Röntgenstrukturanalyse bestätigt.

¹H-NMR (400 MHz, CDCl₃, δ [ppm]): 7,11-6,84 (m, 5H, ArH), 0,12 (s, 9H, SiMe3). ¹³C-NMR (101 MHz, CDCl₃, δ [ppm]): 157,1 (CO), 141,6 (ArC), 128,7 (ArCH), 126,5 (ArCH), 119,5-116,1 (ArCH), 0,7 (SiMe3). ²⁹Si-NMR (79 MHz, CDCl₃, δ [ppm]): 23,4, 10,7. Elementaranalyse: berechnet N 4,98 %, C 55,47 %, H 8,24 %; gemessen N 5.07 %, C 55,01 %, H 8,215 %.

### Stufe 4: Thermolyse

Einige Gramm *N*,*O*-Bis(trimethylsilyl)-*N*-phenyl-carbamat (**1**) wurden unter inerten Bedingungen in die Sumpf-Kugel der Kugelrohrdestille gefüllt. Die Sumpf-Kugel sowie die mittlere Kugel wurden in den Heizraum (Röhrenofen) geschoben, während die äußere Kugel im Wasserbad gekühlt wurde. Bei einer Drehung von 20 Umdrehungen pro Minute (U/min) wurde ein Temperaturprofil durchlaufen. Der Ofen wurde für 30 Minuten auf 170 °C erwärmt, anschließend wurde die Temperatur für weitere 30 Minuten auf 200 °C erhöht und abschließend für ebenfalls 30 min auf 230 °C erhitzt. Nach dem Schmelzen des eingesetzten Feststoffes konnte eine Ansammlung von farbloser Flüssigkeit sowohl in der mittleren als auch in der äußeren Kugel beobachtet werden. Die Kugeln wurden nach Ablauf der Reaktionszeit auf Raumtemperatur abgekühlt und inert in Schlenk-Gefäße überführt. Die Untersuchung der Zusammensetzung erfolgte mittels NMR-Spektroskopie. Während in der mittleren Kugel noch unzersetztes Carbamat vorzufinden war, zeigte sich im ²⁹Si-NMR-Spektrum der äußeren Kugel nur die Anwesenheit von Hexamethyldisiloxan. Diese Ergebnisse werden von den ¹³C-NMR-Spektren bestätigt. Zudem findet sich in den ¹³C-NMR-Spektren der mittleren und äußeren Kugel jeweils ein Signal bei 134,0 ppm, welches eindeutig dem Phenyl-Isocyanat zugeordnet werden kann. Während in der mittleren Kugel eine Vielzahl weiterer Signale auf ein Stoffgemisch verschiedener Spezies hindeutet, findet sich in der Probe der äußeren Kugel nur das Isocyanat zusammen mit Hexamethyldisiloxan (¹³C: δ = 2,0 ppm). Ferner wurde die Anwesenheit des Isocyanates mittels IR-ATR-Spektroskopie überprüft. Auch hier konnte eindeutig die Isocyanat-Bande bei 2258 cm⁻¹ identifiziert werden. Der Vergleich beider Analysen mit einer kommerziell erhältlichen Probe des Phenyl-Isocyanates lieferte exakte Übereinstimmungen.

### Beispiel 2

### Synthese von n-Octyl-isocyanat (12)

*n*-Octyl-isocyanat (Verbindung **12**) wurde gemäß den in Schema 11 gezeigten Verfahren hergestellt. *n*-Octyl-isocyanat ist ein Beispiel eines aliphatischen Monoisocyanats.

### Stufe 1: Aminosilansvnthese

6,02 g (46,56 mmol) *n*-Octylamin wurden zusammen mit 4,79 g (47,31 mmol) Triethylamin in 100 ml Diethylether vorgelegt. Unter Rühren im Eisbad erfolgte die Zugabe von 5,04 g (46,39 mmol) Trimethylchlorsilan über einen Tropftrichter. Beim Zutropfen des Silans konnte die Bildung eines wei-ßen Feststoffes sowie eine weiße Rauchentwicklung beobachtet werden. Anschließend wurde das Gemisch auf Raumtemperatur erwärmt und für 24 Stunden gerührt. Nach Entfernen des weißen Feststoffes mittels Filtration sowie Entfernen des Lösungsmittels unter vermindertem Druck konnten mit einer Ausbeute von 87 % 8,11 g (40,24 mmol) N-Trimethylsilyl-*n*-octylamin als klare, farblose Flüssigkeit erhalten werden. Die Reinheit des Produktes wurde mittels NMR-Spektroskopie bestätigt.

¹H-NMR (400 MHz, CDCl₃, δ [ppm]): 2,81 (t, 2H, CH₂), 1,50-1,40 (m, 12H, CH₂), 1,01 (t, 3H, CH₃), 0,49 (s, 1H, NH), 0,15 (s, 9H, SiMe3). ¹³C-NMR (101 MHz, CDCl₃, δ [ppm]): 42,0 (CH₂), 35,0 (CH₂), 32,1 (CH₂), 29,7 (CH₂), 29,5 (CH₂), 27,1 (CH₂), 22,8 (CH₂), 14,1 (CH₃), 0,0 (SiMe3). ²⁹Si-NMR (79 MHz, CDCl₃, δ [ppm]): 3,0.

### Stufe 2: CO₂-Insertion

8,11 g (40,24 mmol) *N*-Trimethylsilyl-*n*-octylamin wurden mit 44 ml trockenem THF vermengt und im Eisbad gekühlt. Für 30 Minuten erfolgte die Gaseinleitung von CO₂ unter konstantem Rühren des Reaktionsgemisches. Anschließend wurde das Lösungsmittel in einer Kältedestillation entfernt und das Insertionsprodukt als klare, farblose Flüssigkeit gewonnen. Es konnten 8,68 g (35,35 mmol) *N*,*n*-Octyl-*O*-trimethylsilyl-carbamat erhalten werden, was einer Ausbeute von 88 % entspricht. Die Identität und Reinheit der Zielverbindung wurden mittels NMR-Spektroskopie bestätigt.

¹H-NMR (400 MHz, CDCl₃, δ [ppm]): 5,54 (s, 1H, NH), 3,10 (t, 2H, CH₂), 1,49-1,29 (m, 12H, CH₂), 0,88 (t, 3H, CH₃), 0,26 (s, 9H, SiMe3). ¹³C-NMR (101 MHz, CDCl₃, δ [ppm]): 155,5 (CO), 41,1 (CH₂), 31,9 (CH₂), 30,1 (CH₂), 29,4 (CH₂), 27,0 (CH₂), 25,7 (CH₂), 22,7 (CH₂), 14,1 (CH₃), 0,0 (SiMe3). ²⁹Si-NMR (79 MHz, CDCl₃, δ [ppm]): 21,7.

### Stufe 3: Silylierung

8,68 g (35,35 mmol) *N*,*n*-Octyl-*O*-trimethylsilyl-carbamat wurden zusammen mit 3,64 g (35,99 mmol) Triethylamin in 100 ml n-Pentan gelöst und im Eisbad gerührt. Über einen Tropftrichter wurden 8,05 g (36,23 mmol) Trimethylsilyltriflat zugetropft. Es konnte die Bildung einer zweiten Phase am Boden des Schlenkkolbens beobachtet werden. Das Gemisch wurde anschließend für 3 Stunden bei Raumtemperatur gerührt. Die entstandene 2. Phase wurde im Trockeneis/Isopropanol-Kältegemisch zum Erstarren gebracht und die überstehende Lösung abdekantiert. In einer Kältedestillation wurde das Lösungsmittel entfernt, wobei eine schwach gelbe, klare Flüssigkeit im Reaktionskolben verbleibt. Auf diese Weise konnten 8,83 g (27,81 mmol) *N*,*O*-Bis(trimethylsilyl)-*N*,*n*-octyl-carbamat (**2**) erhalten werden. Dies entspricht einer Ausbeute von 79 %. Die Identität und Reinheit des Zielproduktes wurde mittels NMR-Spektroskopie bestätigt.

¹H-NMR (400 MHz, CDCl₃, δ [ppm]): 5,37 (s, 1H, NH), 3,01 (t, 2H, CH₂), 1,38-1,20 (m, 12H, CH₂), 0,80 (t, 3H, CH₃), 0,20 (s, 9H, SiMe3), 0,14 (s, 9H, SiMe3). ¹³C-NMR (101 MHz, CDCl₃, δ [ppm]): 157,6 (CO), 44,8 (CH₂), 32,0 (CH₂), 31,1 (CH₂), 29,7 (CH₂), 29,5 (CH₂), 27,2 (CH₂), 22,8 (CH₂), 14,2 (CH₃), 0,8 (SiMe3), 0,0 (SiMe3). ²⁹Si-NMR (79 MHz, CDCl₃, δ [ppm]): 21,3, 8,8.

### Stufe 4: Thermolyse

Einige Milliliter *N*,*O*-Bis(trimethylsilyl)-*N*,*n*-octyl-carbamat (**2**) wurden unter inerten Bedingungen in die Sumpf-Kugel der Kugelrohrdestille gefüllt. Die Sumpf-Kugel sowie die mittlere Kugel wurden in den Heizraum (Röhrenofen) geschoben, während die äußere Kugel im Wasserbad gekühlt wurde. Das Carbamat wurde für 2 Stunden auf 300 °C erhitzt, während die Kugelrohrdestille kontinuierlich mit 20 U/min gedreht wurde. In der äußeren Kugel sammelte sich eine farblose Flüssigkeit an. Die mittlere Kugel enthielt am Ende der Reaktionszeit eine gelbe Flüssigkeit, und die Sumpf-Kugel war nahezu leer. Die Kugeln wurden anschließend auf Raumtemperatur abgekühlt und inert in Schlenk-Gefäße überführt. Die Untersuchung der Zusammensetzung erfolgte mittels NMR-Spektroskopie. Während in der mittleren Kugel noch unzersetztes Carbamat vorzufinden war, zeigte sich im ²⁹Si-NMR-Spektrum der äußeren Kugel nur die Anwesenheit von Hexamethyldisiloxan. Diese Ergebnisse werden von den ¹³C-NMR-Spektren bestätigt. Zudem findet sich in dem ¹³C-NMR-Spektrum der äußeren Kugel jeweils ein Signal bei 120,5 ppm, welches eindeutig dem n-Octyl-isocyanat zugeordnet werden kann. Während in der mittleren Kugel eine Vielzahl weiterer Signale auf ein Stoffgemisch verschiedener Spezies hindeutet, findet sich in der Probe der äußeren Kugel nur das Isocyanat zusammen mit Hexamethyldisiloxan (¹³C: δ = 2,0 ppm). Der Vergleich der Analyse mit einer kommerziell erhältlichen Probe des n-Octyl-Isocyanates lieferte exakte Übereinstimmungen.

### Beispiel 3

### Synthese von 1,8-Diisocyanatooctan (16)

1,8-Diisocyanatooctan (Verbindung 16) wurde gemäß den in Schema 12 gezeigten Verfahren hergestellt. 1,8-Diisocyanatooctan ist ein Beispiel eines aliphatischen Diisocyanats.

### Stufe 1: Aminosilansvnthese

3,10 g (21,52 mmol) 1,8-Diaminooctan wurden zusammen mit 5,12 g (50,63 mmol) Triethylamin in 140 ml Diethylether vorgelegt. Unter Rühren im Eisbad erfolgte die Zugabe von 4,99 g (45,97 mmol) Trimethylchlorsilan über einen Tropftrichter. Beim Zutropfen des Silans konnte die Bildung eines weißen Feststoffes sowie eine weiße Rauchentwicklung beobachtet werden. Nach vollständiger Zugabe des Chlorsilans wurde das Gemisch über Nacht bei Raumtemperatur gerührt. Nach Entfernen des weißen Feststoffes durch Filtration sowie Entfernen des Lösungsmittels unter vermindertem Druck konnten das Aminosilan als farblose Flüssigkeit gewonnen werden. Auf diese Weise konnten 4,71 g (16,33 mmol) *N*,*N*'-Bis(trimethylsilyl)-1,8-diaminooctan erhalten werden. Die Ausbeute beträgt 76 %. Die Reinheit des Produktes wurde mittels NMR-Spektroskopie bestätigt.

¹H-NMR (400 MHz, CDCl₃, δ [ppm]): 2,81 (m, 4H, CH₂), 1,49-1,41 (m, 12H, CH₂), 0,45 (s, 2H, NH), 0,15 (s, 18H, SiMe3). ¹³C-NMR (101 MHz, CDCl₃, δ [ppm]): 41,9 (CH₂), 34,9 (CH₂), 29,6 (CH₂), 26,9 (CH₂), 0,0 (SiMe3). ²⁹Si-NMR (79 MHz, CDCl₃, δ [ppm]): 2,9.

### Stufe 2: CO₂-Insertion

7,04 g (24,39 mmol) N,N`-Bis(trimethylsilyl)-1,8-diaminooctan wurden mit 40 ml trockenem THF vermengt. Die COz-Insertion erfolgte mittels Gaseinleitung für 20 Minuten, während das Reaktionsgemisch gerührt und im Eisbad gekühlt wurde. Das Lösungsmittel wurde unter vermindertem Druck entfernt und der erhaltene, weiße Feststoff im Vakuum getrocknet. Auf diesem Weg konnten 8,52 g (22,62 mmol) 1,8-[O, O '-Bis(trimethylsilyl)-carbamato]-octan in einer Ausbeute von 93 % erhalten werden. Die Identität und Reinheit der Zielverbindung wurden mittels NMR-Spektroskopie bestätigt.

¹H-NMR (400 MHz, CDCl₃, δ [ppm]): 6,05 (s, 2H, NH), 3,06 (m, 4H, CH₂), 1,46-1,31 (m, 12H, CH₂), 0,08 (s, 18H, SiMe3). ¹³C-NMR (101 MHz, CDCl₃, δ [ppm]): 155,6 (CO), 41,2 (CH₂), 30,2 (CH₂), 29,6 (CH₂), 27,1 (CH₂), 0,0 (SiMe3). ²⁹Si-NMR (79 MHz, CDCl₃, δ [ppm]): 20,9.

### Stufe 3: Silylierung

6,22 g (16,53 mmol) 1,8-[O,O`-Bis(trimethylsilyl)-carbamato]-octan wurden zusammen mit 3,37 g (33,34 mmol) Triethylamin in 50 ml Anisol gelöst. Das Gemisch wurde im Eisbad gerührt. Über einen Tropftrichter wurden 7,38 g (33,19 mmol) Trimethylsilyltriflat zugetropft. Es konnte die Bildung einer flockenartigen zweiten Phase beobachtet werden. Das Gemisch wurde anschließend auf Raumtemperatur erwärmt und für 2 Stunden bei dieser Temperatur gerührt. Die entstandene zweite Phase wurde in einem Kältegemisch aus Trockeneis und Isopropanol zum Erstarren gebracht und die überstehende Lösung abdekantiert. Das Lösungsmittel wurde unter reduziertem Druck entfernt, wobei das Ausfallen eines weißen Feststoffes beobachtet werden konnte. Der Feststoff wurde für 4 Stunden im Vakuum getrocknet. Auf diese Weise konnten 8,12 g (15,58 mmol) 1.8-[*N*,*N*',*O*,*O*'-Tetrakis(trimethylsilyl)-carbamato]-octan (**6**) erhalten werden, was einer Ausbeute von 94 % entspricht. Die Identität und Reinheit des Zielproduktes wurden mittels NMR-Spektroskopie bestätigt.

¹H-NMR (400 MHz, CDCl₃, δ [ppm]): 8,78 (s, 2H, NH), 3,07 (m, 4H, CH₂), 1,44-1,26 (m, 12H, CH₂), 0,29 (s, 18H, SiMe3), 0,22 (s, 18H, SiMe3). ¹³C-NMR (101 MHz, CDCl₃, δ [ppm]): 157,1 (CO), 44,8 (CH₂), 30,9 (CH₂), 29,5 (CH₂), 27,0 (CH₂), 0,8 (SiMe3), 0,0 (SiMe3). ²⁹Si-NMR (79 MHz, CDCl₃, δ [ppm]): 22,0, 9,5.

### Stufe 4: Thermolyse

Einige Gramm 1,8-[*N*,*N*',*O*,*O'*-Tetrakis(trimethylsilyl)-carbamato]-octan (6) wurden in die Sumpfkugel der Kugelrohrdestille unter inerten Bedingungen eingefüllt. Die Sumpf-Kugel sowie die mittlere Kugel wurden in den Heizraum (Röhrenofen) geschoben, während die äußere Kugel im Wasserbad gekühlt wurde. Das Carbamat wurde für 1 Stunde auf 170 °C, 1 Stunde auf 200 °C und 30 min auf 250 °C erhitzt, während die Kugelrohrdestille kontinuierlich mit 30 U/min gedreht wurde. Zum Ende der Heizphase konnte eine Ansammlung von farbloser Flüssigkeit in der äußeren Kugel beobachtet werden, während sich in der Sumpf-Kugel eine gelbe, polymerartige Schicht gebildet hat. Nach erfolgter Thermolyse wurden die einzelnen Kugeln der Kugelrohrdestille separat mittels NMR-Spektroskopie untersucht. Das Vorliegen des Diisocyanates konnte eindeutig über NMR- und IR-Spektroskopie nachgewiesen werden.

Das ¹³C-NMR-Spektrum der äußeren Kugel zeigt neben Hexamethyldisiloxan (¹³C: δ = 2,0 ppm, ²⁹Si: δ = 7,1 ppm) das Diisocyanat bei einer Verschiebung von 120,5 ppm. Die zugehörige Bande im IR-ATR-Spektrum liegt bei 2253 cm⁻¹. Der Vergleich mit einer kommerziellen Probe von 1,8-Diisocyanatooctan liefert eine vollständige Übereinstimmung.

### Beispiel 4

### Synthese von Isopentvl-isocvanat (21)

*i*-Pentyl-isocyanat (Verbindung **21**) wurde gemäß den in Schema 11 gezeigten Verfahren hergestellt. *i*-Pentyl-isocyanat ist ein weiteres Beispiel eines aliphatischen Monoisocyanats. Bezogen auf Schema 11 sind in Beispiel 4 R¹ i-Pentyl, R² und R³ bei jedem Auftreten Methyl, X¹ Chlor und X Triflat. Unter der Angabe "i-Pentyl" oder "Isopentyl" wird eine Gruppe der Formel (H₃C)₂CH-CH₂-CH₂- verstanden.

*N*,*O*-Bis(trimcthylsilyl)-*N*,*i*-pentyl-carbamat (**22**) ist eine Verbindung der allgemeinen Formel IV-A, in der R¹ i-Pentyl ist. N,*O*-Bis(trimethylsilyl)-*N*,*i-*pentyl-carbamat (**22**) weist folgende Formel auf:

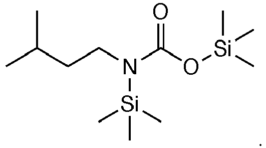

### Stufe 1: Aminosilansvnthese

5,65 g (64,81 mmol) i-Pentylamin wurden zusammen mit 9,81 g (96,99 mmol) Triethylamin in 150 ml Diethylether vorgelegt. Unter Rühren im Eisbad erfolgte die Zugabe von 9,81 g (90,32 mmol) Trimethylchlorsilan über einen Tropftrichter. Beim Zutropfen des Silans konnte neben der Bildung eines weißen Feststoffes eine weiße Rauchentwicklung beobachtet werden. Anschließend wurde das Gemisch auf Raumtemperatur erwärmt und für 96 Stunden gerührt. Der Feststoff wurde über eine Filtration abgetrennt und das Lösungsmittel unter vermindertem Druck entfernt. Das Aminosilan *N*-Trimethylsilyl-*i*-pentylamin wurde als farblose Flüssigkeit in einer Ausbeute von 88 % (9,12 g, 57,25 mmol) erhalten. Die Reinheit des Produktes wurde mittels NMR-Spektroskopie bestätigt.

¹H-NMR (500 MHz, CDCl₃, δ [ppm]): 2,69 (t, J = 7,4 Hz, 2H), 1,60 (dq, J = 13,4, 6,7 Hz, 1H), 1,29 - 1,21 (m, 2H), 0.85 (d, J = 6,7 Hz, 6H), 0,24 (s, 1H, NH), 0,00 (s, 9H, SiMe₃). ¹³C-NMR (101 MHz, CDCl₃, δ [ppm]): 44,2 (CH₂), 39,9 (CH₂), 25,5 (CH), 22,6 (CH₃), -0,1 (SiMe₃). ²⁹Si-NMR (79 MHz, CDCl₃, δ [ppm]): 3,0.

### Stufe 2: COz-Insertion

7,99 g (50,11 mmol) *N*-Trimethylsilyl-i-pentylamin wurden mit 10 ml trockenem THF vermengt und unter inerten Bedingungen in einen Autoklav überführt. Der Autoklav wurde mit 8 bar CO₂-Druck beaufschlagt. Die Reaktion erfolgte unter konstantem Rühren für eine Zeitspanne von 3 Stunden. Nach Ablassen des Druckes und inertem Überführen des Reaktionsgemisches in ein Schlenk-Gefäß wurde das Lösungsmittel in einer Kältedestillation entfernt und das Insertionsprodukt als klare, farblose Flüssigkeit gewonnen. Es konnten 9,55 g (46,98 mmol) *N*,*i*-Pentyl-*O*-trimethylsilyl-carbamat erhalten werden. Dies entspricht einer Ausbeute von 94 %. Die Identität und Reinheit der Zielverbindung wurden mittels NMR-Spektroskopie bestätigt.

¹H-NMR (500 MHz, CDCl₃, δ [ppm]): 5,19 (t, J = 6,1 Hz, 1H, NH), 2,92 - 2,81 (m, 2H), 1,36 (dp, J = 13,3, 6,7 Hz, 1H), 1,17 - 1,06 (m, 2H), 0,65 (d, J = 6,7 Hz, 6H), 0,00 (s, 9H, SiMe₃). ¹³C-NMR (101 MHz, CDCl₃, δ [ppm]): 155,1 (CO), 40,1 (CH₂), 39,0 (CH₂), 25,5 (CH), 22,2 (CH₃), -0,3 (SiMe₃). ²⁹Si-NMR (79 MHz, CDCl₃, δ [ppm]): 21,8.

### Stufe 3: Silylierung

9,55 g (46,98 mmol) *N*,*i*-Pentyl-*O*-trimethylsilyl-carbamat wurden zusammen mit 5,07 g (50,06 mmol) Triethylamin in 90 ml n-Pentan gelöst und im Eisbad gerührt. Über einen Tropftrichter wurden 11,02 g (49,59 mmol) Trimethylsilyltriflat zugetropft, wobei die Bildung einer weißen, zweiten Phase am Boden des Schlenkkolbens beobachtet werden konnte. Das Gemisch wurde anschließend über Nacht bei Raumtemperatur gerührt. Die entstandene 2. Phase wurde im Trockeneis/Isopropanol-Kältegemisch zum Erstarren gebracht und die überstehende Lösung abdekantiert. Das Lösungsmittel wurde mittels Kältedestillation entfernt. Auf diese Weise konnten 8,38 g (30,40 mmol, Ausbeute 65 %) *N,O*-Bis(trimethylsilyl)-*N,i*-pentyl-carbamat (**22**) als schwach gelbe Flüssigkeit erhalten werden. Die Identität des Zielproduktes wurde mittels NMR-Spektroskopie bestätigt.

¹H-NMR (500 MHz, CDCl₃, δ [ppm]): 2,93 - 2,80 (m, 2H), 1,31 (dh, J = 13,3, 6,7 Hz, 1H), 1,13 (ddd, J = 11,7, 7,6, 5,9 Hz, 2H), 0,67 (d, J = 6,7 Hz, 6H), 0,06 (s, 9H, SiMe₃), 0,00 (s, 9H, SiMe₃). ¹³C-NMR (101 MHz, CDCl₃, δ [ppm]): 157,3 (CO), 43,0 (CH₂), 39,6 (CH₂), 26,2 (CH), 22,4 (CH₃), 0,5 (SiMe₃), -0,3 (SiMe₃). ²⁹Si-NMR (79 MHz, CDCl₃, δ [ppm]): 21,9 (Si-O), 9,5 (Si-N).

### Stufe 4: Thermolyse

1,38 g (4,99 mmol) *N*,*O*-Bis(trimethylsilyl)-*N*,*i*-pentyl-carbamat (**22**) wurden unter inerten Bedingungen in die Sumpf-Kugel der Kugelrohrdestille gefüllt. Die Sumpf-Kugel sowie die mittlere Kugel wurden in den Heizraum (Röhrenofen) geschoben und die Apparatur auf eine Neigung von 45° gestellt. Das Carbamat wurde bei kontinuierlicher Drehung von 30 U/min für 1 Stunde auf 250 °C und weitere 30 min auf 300 °C erhitzt. Anschließend wurde die Apparatur in eine waagerechte Position gebracht und erneut für 30 min auf 300 °C geheizt. In der äußeren und mittleren Kugel sammelte sich eine farblose Flüssigkeit an. Die Sumpf-Kugel war am Ende der Reaktionszeit nahezu leer. Die Kugeln wurden anschließend auf Raumtemperatur abgekühlt und inert in Schlenk-Gefäße überführt. Die Untersuchung der farblosen Flüssigkeit der äußeren Kugel erfolgte mittels NMR-Spektroskopie. Das ²⁹Si-NMR-Spektrum zeigt nur die Anwesenheit von Hexamethyldisiloxan (7,1 ppm). Das eingesetzte Carbamat ist vollständig zersetzt. Diese Ergebnisse werden von dem ¹³C-NMR-Spektrum bestätigt (Hexamethyldisiloxan bei 1,7 ppm). Zudem findet sich in dem ¹³C-NMR-Spektrum ein Signal bei 122,2 ppm, welches eindeutig dem Isopentyl-isocyanat zugeordnet werden kann (weitere zugehörige Signale: 41,0, 40,1, 25,2, 21,9 ppm). Die Zuordnung wurde mittels IR-Spektroskopie zusätzlich bestätigt. Die charakteristische Schwingung der Carbonylgruppe des Carbamats bei rund 1690 cm⁻¹ ist vollständig verschwunden, während bei 2270 cm⁻¹ die intensive Isocyanat-Bande zu finden ist. Die Banden bei 1252, 1053, 841 cm⁻¹ können dem Hexamethyldisiloxan zugeordnet werden.

### Beispiel 5

### Synthese von Benzyl-isocyanat (23)

Benzyl-isocyanat (Verbindung **23**) wurde gemäß den in Schema 11 gezeigten Verfahren hergestellt. Benzyl-isocyanat ist ein weiteres Beispiel eines aliphatischen Monoisocyanats. Bezogen auf Schema 11 sind in Beispiel 5 R¹ Benzyl, R² und R³ bei jedem Auftreten Methyl, X¹ Chlor und X Triflat. Unter der Angabe "Benzyl" oder wird eine Gruppe der Formel H₅C₆-CH₂- verstanden.

### Stufe 1: Aminosilansvnthese

5,03 g (46,90 mmol) Benzylamin wurden zusammen mit 4,91 g (48,55 mmol) Triethylamin in 160 ml Diethylether vorgelegt. Unter Rühren im Eisbad erfolgte die Zugabe von 5,17 g (47,56 mmol) Trimethylchlorsilan über einen Tropftrichter, wobei die Bildung eines weißen Feststoffes sowie eine weiße Rauchentwicklung beobachtet werden konnten. Anschließend wurde das Gemisch auf Raumtemperatur erwärmt und über Nacht gerührt. Der Feststoff wurde über eine Filtration abgetrennt und das Lösungsmittel mittels Kältedestillation entfernt. Das Aminosilan N-Trimethylsilyl-benzylamin wurde als farblose Flüssigkeit in einer Ausbeute von 89 % (7,45 g, 41,52 mmol) erhalten. Die Reinheit des Produktes wurde mittels NMR-Spektroskopie bestätigt.

¹H-NMR (500 MHz, CDCl₃, δ [ppm]): 7,15 (m, 4H), 7,05 (m, 1H), 3,77 (d, J = 8,0 Hz, 2H), 0,55 (s, 1H, NH), 0,00 (s, 9H, SiMe₃). ¹³C-NMR (101 MHz, CDCl₃, δ [ppm]): 144,2 (C), 128,1(CH), 126,8 (CH), 126,3 (CH), 45,6 (CH2), 0,0 (SiMe₃). ²⁹Si-NMR (79 MHz, CDCl₃, δ [ppm]): 3,9.

### Stufe 2: CO₂-Insertion

6,78 g (37,81 mmol) N Trimethylsilyl-benzylamin wurden mit 20 ml trockenem THF vermengt, unter inerten Bedingungen in einen Autoklav überführt und eine Stunde mit 8 bar CO₂-Druck beaufschlagt. Nach Ablassen des Druckes und inertem Überführen des Reaktionsgemisches in ein Schlenk-Gefäß wurde das Lösungsmittel in einer Kältedestillation entfernt und das Insertionsprodukt als farblose, viskose Flüssigkeit mit einer Ausbeute von 94 % (7,90 g, 35,37 mmol) gewonnen. Nach einigen Tagen Lagerung bei Raumtemperatur konnte die Bildung von farblosen Kristallen beobachtet werden. Die Identität und Reinheit der Zielverbindung wurden mittels NMR-Spektroskopie bestätigt.

¹H-NMR (500 MHz, CDCl₃, δ [ppm]): 7,14 (m, 5H), 6,17 (s, 1H, NH), 4,15 (d, J = 6,2 Hz, 2H, CH₂), 0,23 (s, 9H, SiMe₃). ¹³C-NMR (101 MHz, CDCl₃, δ [ppm]): 155,0 (CO), 128,6 (C), 127,8 (CH), 126,8 (CH), 126,5 (CH), 44,2 (CH2), -0,5 (SiMe₃). ²⁹Si-NMR (79 MHz, CDCl₃, δ [ppm]): 23,2. Elementaranalyse: berechnet N 6,27 %, C 59,16 %, H 7,67 %, gemessen N 6,81 %, C 59,02 %, H 7,408 %

### Stufe 3: Silylierung

30,33 g (135,80 mmol) N-Benzyl-O-trimethylsilyl-carbamat wurden zusammen mit 14,48 g (143,07 mmol) Triethylamin in 100 ml Diethylether gelöst und im Eisbad gerührt. Über einen Tropftrichter wurden 32,62 g (146,76 mmol) Trimethylsilyltriflat zugetropft, wobei die Bildung einer wei-ßen, zweiten Phase am Boden des Schlenkkolbens beobachtet werden konnte. Das Gemisch wurde anschließend über Nacht bei Raumtemperatur gerührt, bevor die entstandene 2. Phase im Trockeneis/Isopropanol-Kältegemisch zum Erstarren gebracht und die überstehende Lösung abdekantiert wurde. Das Lösungsmittel wurde mittels Kältedestillation aus der Reaktionslösung entfernt und lieferte das Zielprodukt, *N*,*O*-Bis(trimethylsilyl)-*N*-benzyl-carbamat (**5**), als intensiv gelbe, leicht viskose Flüssigkeit (37,58 g, 127,17 mmol, 94% Ausbeute). Die Identität des Zielproduktes wurde mittels NMR-Spektroskopie bestätigt.

¹H-NMR (500 MHz, CDCl₃, δ [ppm]): 7,18 (m, 5H), 4,41 (s, 2H, CH₂), 0,33 (s, 9H, SiMe₃), 0,19 (s, 9H, SiMe₃). ¹³C-NMR (101 MHz, CDCl₃, δ [ppm]): 158,2 (CO), 140,4 (C), 128,4 (CH), 126,6 (CH), 126,4 (CH), 48,1 (CH₂), 0,8 (SiMe₃), 0,0 (SiMe₃). ²⁹Si-NMR (79 MHz, CDCl₃, δ [ppm]): 22,6 (O-Si), 10,9 (N-Si).

### Stufe 4: Thermolyse

3,13 g (4,50 mmol) *N,O*-Bis(trimethylsilyl)-N-benzyl-carbamat (**5**) wurden unter inerten Bedingungen in die Sumpf-Kugel der Kugelrohrdestille gefüllt. Die Sumpf-Kugel sowie die mittlere Kugel wurden in den Heizraum (Röhrenofen) geschoben und die Apparatur auf eine Neigung von 45° gestellt. Das Carbamat wurde bei kontinuierlicher Drehung von 20 U/min für je 30 Minuten auf 200 °C und 250 °C, 10 Minuten 300 °C und weitere 90 Minuten auf 250 °C erhitzt. Der Druckausgleich war über eine Ölfalle gewährleistet. 50 Minuten nach Heizbeginn wurde die Apparatur in eine waagerechte Position gebracht. Ab 250 °C sammelte sich in der äußeren eine farblose Flüssigkeit (1,82 g) und in der mittleren Kugel eine gelbe Flüssigkeit (0,38 g) an. Die Sumpf-Kugel enthielt am Ende der Reaktionszeit geringe Mengen eines hochviskosen, dunkelbraunen Rückstandes (0,64 g). Die Kugeln wurden anschließend auf Raumtemperatur abgekühlt und inert mit CDCl₃ als Lösungsmittel in Schlenk-Gefäße überführt. Die Untersuchung der Fraktionen erfolgte mittels NMR-Spektroskopie.

Äußere Kugel: Das ²⁹Si-NMR-Spektrum zeigte nur die Anwesenheit von Hexamethyldisiloxan (7,3 ppm). Das eingesetzte Carbamat war vollständig zersetzt. Diese Ergebnisse wurden von dem ¹³C-NMR-Spektrum bestätigt (Hexamethyldisiloxan bei 1,7 ppm). Zudem fanden sich in dem ¹³C-NMR-Spektrum Signale, welche eindeutig dem Benzyl-isocyanat durch Vergleich mit einer kommerziell erhältlichen Probe zugeordnet werden können (137,0, 128,7, 127,8, 126,6, 123,7, 46,4 ppm). Sowohl die mittlere Fraktion, als auch der Rückstand wiesen eine Vielzahl von Signalen im ²⁹Si- und ¹³C-NMR Spektrum auf. Eine Zuordnung konnte nicht vorgenommen werden. Eine Untersuchung mittels Gaschromatographie liefert weitere Aufschlüsse über die Zusammensetzung der äußeren Fraktion: Retentionszeit (RT) 1,565 min - 16,95 % - CDCl₃; RT 1,771 min - 0,68 % - Me₃Si-isocyanat (wobei Me eine Methylgruppe bezeichnet); RT 2,043 min - 64,85 % - Hexamethyldisiloxan (HMDSO); RT 2,554 min - 3,67 % - Toluol; RT 6,447 min - 9,48 % - Benzyl-isocyanat (23); RT 10,765 min - 0,81 % - *N*,*O*-Bis(trimethylsilyl)-*N*-benzyl-carbamat (**5**).

In einem weiteren Experiment wurden 8,38 g (28,36 mmol) *N*,*O*-Bis(trimethylsilyl)-*N*-benzyl-carbamat (5) direkt auf 300 °C für 2,5 Stunden in der Kugelrohrdestille bei 30 U/min unter Normaldruck erhitzt. Die erste Stunde war die Apparatur auf eine Neigung von 45 ° gestellt. Nach Ende der Reaktionszeit hatte sich eine farblose Flüssigkeit mit weißen Flocken in der äußeren Kugel (1,74 g) gesammelt, während die mittlere Kugel nahezu leer war (0,22 g) und sich ein hochviskoser, tiefbrauner Rückstand (2,47 g) in der Sumpf-Kugel gebildet hatte. Alle Fraktionen wurden mittels NMR-Spektroskopie untersucht. Erneut zeigten sich eine Vielzahl von Signalen im ²⁹Si- und ¹³C-NMR Spektrum der mittleren Kugel und des Rückstandes, welche nicht weiter zugeordnet werden konnten. Die äußere Kugel enthielt neben HMDSO (7,3 ppm) noch Trimethylsilyl-isocyanat (4,2 ppm) als siliciumhaltige Komponenten im ²⁹Si-NMR Spektrum. Dies bestätigte sich im ¹³C-NMR Spektrum: HMDSO (1,9 ppm), Me₃SiNCO (0,9, 124,0 ppm). Zusätzlich fiel auf, dass die CH₂-Brücke der Benzyl-Gruppe bei rund 46 ppm nicht vorhanden war, sich aber stattdessen ein intensives Signal bei 21,5 ppm zeigte. Zusammen mit den Signalen im aromatischen Verschiebungsbereich konnte die Bildung von Toluol identifiziert werden (137,9, 129,2, 128,4, 125,5, 21,5 ppm).

### Beispiel 6

### Synthese von Allvl-isocvanat (24)

Allyl-isocyanat (Verbindung 2**4**) wurde gemäß den in Schema 11 gezeigten Verfahren hergestellt. Allyl-isocyanat ist ein weiteres Beispiel eines aliphatischen Monoisocyanats. Bezogen auf Schema 11 sind in Beispiel 6 R¹ Allyl, R² und R³ bei jedem Auftreten Methyl, X¹ Chlor und X Triflat. Unter der Angabe "Allyl" wird eine Gruppe der Formel H₂C=CH-CH₂- verstanden.

*N,O*-Bis(trimethylsilyl)-*N*-allyl-carbamat (**25**) ist eine Verbindung der allgemeinen Formel IV-A, in der R¹ Allyl ist. N, O*-*Bis(trimethylsilyl)-*N*-allyl-carbamat (**25**) weist folgende Formel auf:

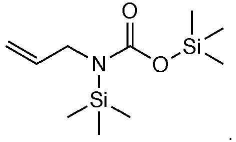

### Stufe 1: Aminosilansvnthese

Zu einem Gemisch aus 5,07 (88,75 mmol) Allylamin und 8,86 g (87,53 mmol) Triethylamin in 100 ml trockenem n-Pentan wurden unter Rühren und Eisbadkühlung 9,73 g (89,59 mmol) Trimethylchlorsilan zugetropft. Es erfolgte die Bildung eines weißen Niederschlages, welcher nach 2-tägigem Rühren bei Raumtemperatur mittels Filtration abgetrennt wurde. Das Lösungsmittel wurde mit Hilfe einer Kältedestillation abgetrennt und der Erfolg der Synthese mittels NMR-Spektroskopie überprüft. Neben dem gewünschten N-Trimethylsilyl-allylamin (**26**) hatte sich das zugehörige Silazan (*N,N-*bis(trimethylsilyl)-allylamin (**27**) gebildet.

¹H-NMR (500 MHz, CDCl₃, δ [ppm]): 5,82 - 5,59 (m, 1H), 4,92 (dd, J = 17,0, 1,8 Hz, 2H), 4,76 (dd, J = 10,1, 1,5 Hz, 1H), 3,26 (dt, J = 4,4, 2,0 Hz, 0H), 3,14 (d, J = 5,3 Hz, 7H), -0,09 (s, 2H), - 0,15 (s, 9H, SiMe₃). ¹³C-NMR (101 MHz, CDCl₃, δ [ppm]): 141,2 (**27**)/140,9 (**26**) (CH), 113,3 (**27**)/112,8 (**26**) (CH2), 47,4 (**27**)/44,6 (**26**) (CH₂), 0,0 (SiMe₃). ²⁹Si-NMR (79 MHz, CDCl₃, δ [ppm]): 4,1 (**26**), 6,5 (**27**).

### Stufe 2: CO₂-Insertion

7,65 g (59,18 mmol) N-Trimethylsilyl-allylamin (kommerziell erworben) wurden mit 20 ml trockenem THF vermengt und unter inerten Bedingungen in einen Autoklav überführt. Die CO₂-Insertionsreaktion erfolgte mit 8 bar CO₂-Druck unter konstantem Rühren für eine Zeitspanne von 43,5 Stunden. Nach Ablassen des Druckes und inertem Überführen des Reaktionsgemisches mit 5 ml THF in ein Schlenk-Gefäß wurde das Lösungsmittel in einer Kältedestillation entfernt und das Insertionsprodukt als intensiv gelbe, leicht viskose Flüssigkeit gewonnen. Auf diesem Weg konnten 9,55 g (55,10 mmol, 93 % Ausbeute) N-Allyl-O-trimethylsilyl-carbamat erhalten werden. Die Identität und Reinheit der Zielverbindung wurden mittels NMR-Spektroskopie bestätigt.

¹H-NMR (500 MHz, CDCl₃, δ [ppm]): 5,70 - 5,45 (m, 1H), 5,29 (s, 1H, NH), 4,93 (dd, J = 17,2, 1,6 Hz, 1H), 4,84 (dd, J = 10,3, 1,5 Hz, 1H), 3,50 (t, J = 5,7 Hz, 2H), 0,04 (s, 9H, SiMe₃). ¹³C-NMR (101 MHz, CDCl₃, δ [ppm]): 155,6 (CO), 135,2 (CH), 115,7 (CH₂), 43,7 (CH₂), 0,3 (SiMe₃). ²⁹Si-NMR (79 MHz, CDCl₃, δ [ppm]): 22,3.

### Stufe 3: Silylierung

8,43 g (48,62 mmol) *N*-Allyl-*O*-trimethylsilyl-carbamat wurden zusammen mit 5,28 g (52,14 mmol) Triethylamin in 100 ml n-Pentan gelöst und im Eisbad gerührt. Über einen Tropftrichter wurden 11,37 g (51,15 mmol) Trimethylsilyltriflat zugetropft, wobei die Bildung einer intensiv orangen, zweiten Phase am Boden des Schlenkkolbens beobachtet werden konnte. Das Gemisch wurde anschließend über Nacht bei Raumtemperatur gerührt. Die entstandene 2. Phase wurde im Trockeneis/Isopropanol-Kältegemisch zum Erstarren gebracht und die überstehende Lösung abdekantiert. Nach Entfernen des Lösungsmittels über eine Kältedestillation konnten 10,24 g (41,70 mmol, Ausbeute 86 %) *N,O*-Bis(trimethylsilyl)-N-allyl-carbamat (**25**) als orange Flüssigkeit erhalten werden. Die Identität des Zielproduktes wurde mittels NMR-Spektroskopie bestätigt.

¹H-NMR (500 MHz, CDCl₃, δ [ppm]): 5,67 - 5,53 (m, 1H), 4,88 (d, J = 1,8 Hz, 2H), 4,86 - 4,80 (m, 2H), 3,56 (d, J = 5,1 Hz, 2H), 0,10 (s, 9H, SiMe₃), 0,04 (s, 9H, SiMe₃). ¹³C-NMR (101 MHz, CDCl₃, δ [ppm]): 157,4 (CO), 136,1 (CH), 114,3 (CH₂), 46,7 (CH₂), 0,6 (SiMe₃), -0,1 (SiMe₃). ²⁹Si-NMR (79 MHz, CDCl₃, δ [ppm]): 22,4 (Si-O), 10,5 (Si-N).

### Stufe 4: Thermolyse

1,09 g (4,43 mmol) *N*,*O*-Bis(trimethylsilyl)-*N*-allyl-carbamat (**25**) wurden unter inerten Bedingungen in die Sumpf-Kugel der Kugelrohrdestille gefüllt und zusammen mit der mittlere Kugel in den Heizraum (Röhrenofen) geschoben. Die Apparatur wurde auf eine Neigung von 45° gestellt. Das Carbamat wurde bei kontinuierlicher Drehung von 30 U/min unter Normaldruck für 1 Stunde auf 250 °C und weitere 30 min auf 300 °C erhitzt. Die Apparatur wurde eine Stunde nach Heizbeginn in die waagerechte Position gebracht. In der äußeren Kugel sammelte sich eine farblose Flüssigkeit an. Während die mittlere Kugel am Ende der Reaktionszeit nahezu leer war, hatte sich in der Sumpf-Kugel ein schwarzer Film an der Glaswand gebildet. Die Kugeln wurden anschließend auf Raumtemperatur abgekühlt und die äußere Fraktion inert in Schlenk-Gefäße überführt, bevor die Zusammensetzung mittels NMR-Spektroskopie untersucht wurde. Das ²⁹Si-NMR-Spektrum zeigte nur die Anwesenheit von Hexamethyldisiloxan (7,2 ppm), während das eingesetzte Carbamat vollständig zersetzt war. Diese Ergebnisse wurden von dem ¹³C-NMR-Spektrum bestätigt (Hexamethyldisiloxan bei 2,0 ppm). Die weiteren Signale im ¹³C-NMR Spektrum zeigten eine genaue Übereinstimmung mit einer Probe kommerziell erhältlichen Allyl-isocyanates. Die Signallagen sind: 133,3, 123,9, 116,1, 39,7 ppm. Die Zuordnung wurde durch die charakteristische Schwingung der Isocyanatgruppe bei 2262 cm⁻¹ im IR-Spektrum zusätzlich bestätigt. Die Gehaltsbestimmung mittels Gaschromatographie ergab eine Zusammensetzung von 66,05 % Hexamethyldisiloxan und 28,38 % Allyl-isocyanat.

## Patentansprüche

1. Verfahren zur Herstellung von Isocyanaten, wobei
(i) eine erste siliciumorganische Verbindung, die zumindest ein Silicium-Atom Si¹ und eine daran gebundene Einheit der Formel G-I 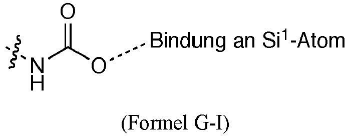 aufweist, durch Silylierung der NH-Gruppe der Einheit der Formel G-I mit einer zweiten siliciumorganischen Verbindung, die ein Silicium-Atom Si² aufweist, in eine dritte siliciumorganische Verbindung überführt wird, die eine Einheit der Formel G-II 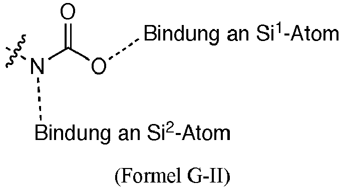 aufweist; und
(ii) die dritte siliciumorganische Verbindung durch Thermolyse zu einem Isocyanat umgesetzt wird, wobei die Einheit der Formel G-II in eine Isocyanat-Gruppe überführt wird.

2. Verfahren nach Anspruch 1, **dadurch gekennzeichnet, dass** die erste siliciumorganische Verbindung eine Verbindung der allgemeinen Formel II 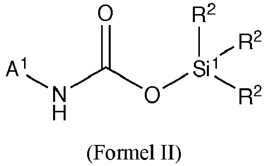 ist, worin
A¹ R¹ oder eine Gruppe der allgemeinen Formel G-III ist: 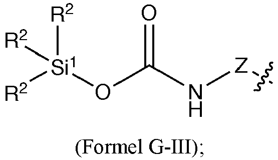
R¹ eine substituierte oder unsubstituierte, aliphatische oder aromatische Gruppe ist;
R² bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si¹ zumindest ein Rest R² gebunden ist, der nicht Wasserstoff ist; und
Z eine substituierte oder unsubstituierte, aliphatische oder aromatische Gruppe ist;
die zweite siliciumorganische Verbindung eine Verbindung der allgemeinen Formel III 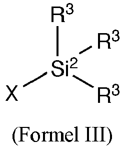 ist, worin
X aus der Gruppe ausgewählt ist, die aus einem Halogen, -CN, -OCN, -SCN, -N₃, einem Sulfonat, einem Carbamat, -O-R⁴, -NR⁷R⁸ oder einem N-Heterocyclus besteht;
R³ bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si² zumindest ein Rest R³ gebunden ist, der nicht Wasserstoff ist;
R⁴ -C(O)R⁹ oder eine Gruppe der allgemeinen Formel G-VI 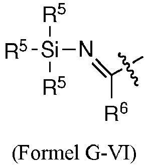 ist,
R⁵ bei jedem Auftreten unabhängig voneinander jeweils eine substituierte oder unsubstituierte Alkylgruppe mit 1 bis 12 Kohlenstoffatomen ist;
R⁶ eine substituierte oder unsubstituierte Alkylgruppe mit 1 bis 12 Kohlenstoffatomen ist;
R⁷ und R⁸ unabhängig voneinander jeweils -C(O)R⁹ oder eine substituierte oder unsubstituierte Alkylgruppe mit 1 bis 12 Kohlenstoffatomen sind; und
R⁹ eine substituierte oder unsubstituierte Alkylgruppe mit 1 bis 12 Kohlenstoffatomen ist; und die dritte siliciumorganische Verbindung eine Verbindung der allgemeinen Formel IV 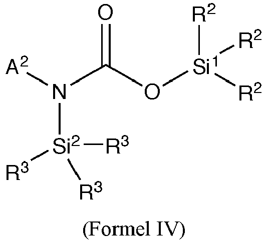 ist, worin
A² R¹ oder eine Gruppe der allgemeinen Formel G-IV ist: 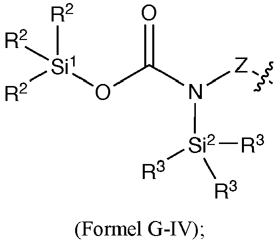
R² bei jedem Auftreten und Z die in Zusammenhang mit der Verbindung der allgemeinen Formel II angegebenen Bedeutungen haben; und
R³ bei jedem Auftreten die in Zusammenhang mit der Verbindung der allgemeinen Formel III angegebene Bedeutungen hat;
wobei die Verbindung der allgemeinen Formel II mit der Verbindung der allgemeinen Formel III zu der Verbindung der allgemeinen Formel IV umgesetzt wird und die Verbindung der allgemeinen Formel IV durch Thermolyse in eine Isocyanat-Verbindung der allgemeinen Formel I 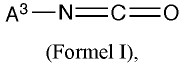
worin
A³ R¹ oder eine Gruppe der Formel G-V ist: 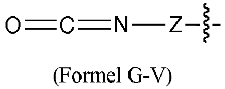
R¹ und Z die in Zusammenhang mit der Verbindung der allgemeinen Formel II angegebenen Bedeutungen haben;
umgesetzt wird.

3. Verfahren nach Anspruch 2, **dadurch gekennzeichnet, dass** R¹ in den Verbindungen der allgemeinen Formel I, II und IV aus der Gruppe ausgewählt ist, die aus einer substituierten oder unsubstituierten Alkylgruppe mit 1 bis 18 Kohlenstoffatomen, einer substituierten oder unsubstituierten Heteroalkylgruppe mit 1 bis 18 Kohlenstoffatomen, einer substituierten oder unsubstituierten Alkenylgruppe mit 1 bis 18 Kohlenstoffatomen, einer substituierten oder unsubstituierten Alkinylgruppe mit 1 bis 18 Kohlenstoffatomen, einer substituierten oder unsubstituierten Arylgruppe und einer substituierten oder unsubstituierten Heteroarylgruppe, besteht.

4. Verfahren nach Anspruch 2 oder Anspruch 3, **dadurch gekennzeichnet, dass** R¹ in den Verbindungen der allgemeinen Formel I, II und IV aus der Gruppe ausgewählt ist, die aus Methyl, Ethyl, Propyl, Butyl, Pentyl, Hexyl, Octyl und Phenyl besteht.

5. Verfahren nach einem der Ansprüche 2 bis 4, **dadurch gekennzeichnet, dass** Z in den Verbindungen der allgemeinen Formel I, II und IV aus der Gruppe ausgewählt ist, die aus einer substituierten oder unsubstituierten Alkylengruppe mit 1 bis 12 Kohlenstoffatomen, einer substituierten oder unsubstituierten Phenylengruppe und einer substituierten oder unsubstituierten Phenylenbisalkylengruppe, in der jede Alkylengruppe bei jedem Auftreten unabhängig voneinander jeweils eine Alkylengruppe mit 1 bis 12 Kohlenstoffatomen ist, besteht.

6. Verfahren nach einem der Ansprüche 2 bis 5, **dadurch gekennzeichnet, dass** X in der Verbindung der allgemeinen Formel III aus der Gruppe ausgewählt ist, die aus Chlor, einer Toluolsulfonsäureester-Gruppe, einer Methylsulfonsäureester-Gruppe und einer Trifluormethylsulfonsäureester-Gruppe besteht.

7. Verfahren nach einem der Ansprüche 2 bis 6, **dadurch gekennzeichnet, dass** R³ bei jedem Auftreten in den Verbindungen der allgemeinen Formel III und IV unabhängig voneinander jeweils eine substituierte oder unsubstituierte Alkylgruppe mit 1 bis 6 Kohlenstoffatomen oder eine substituierte oder unsubstituierte Phenylgruppe ist.

8. Verfahren nach einem der Ansprüche 2 bis 7, **dadurch gekennzeichnet, dass** alle R² in den Verbindungen der allgemeinen Formel I, II und IV jeweils eine Methylgruppe sind und/oder alle R³ in den Verbindungen der allgemeinen Formel III und IV jeweils eine Methylgruppe sind.

9. Verfahren nach einem der vorstehenden Ansprüche, **dadurch gekennzeichnet, dass** die Silylierung bei einer Temperatur von 0 bis 50 °C durchgeführt wird.

10. Verfahren nach einem der vorstehenden Ansprüche, **dadurch gekennzeichnet, dass** die Silylierung in einem unpolaren organischen Lösungsmittel in Gegenwart einer Hilfsbase durchgeführt wird.

11. Verfahren nach einem der vorstehenden Ansprüche, **dadurch gekennzeichnet, dass** die Thermolyse bei einer Temperatur von 150 bis 400 °C durchgeführt wird.

12. Verfahren nach einem der vorstehenden Ansprüche, **dadurch gekennzeichnet, dass** die Thermolyse unter Anwendung eines Temperaturprofils durchgeführt wird, das zwei oder mehr Temperaturstufen umfasst, wobei jede Temperaturstufe für einen vorgegebenen Zeitraum angewendet wird und jede angewendete Temperaturstufe um 20 bis 80 °C über dem zuvor angewendeten Temperaturbereich liegt.

13. Verfahren nach Anspruch 12, **dadurch gekennzeichnet, dass** der vorgegebene Zeitraum zwischen 1 min und 2 h liegt.

14. Verfahren nach einem der vorstehenden Ansprüche, **dadurch gekennzeichnet, dass** die Thermolyse in Gegenwart eines Inertgases durchgeführt wird.

15. Verwendung einer Verbindung der allgemeinen Formel II 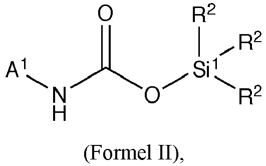 worin
A¹ eine Gruppe der allgemeinen Formel G-III ist: 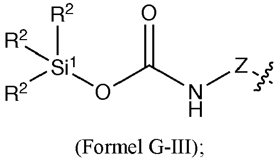
R² bei jedem Auftreten unabhängig voneinander jeweils aus der Gruppe ausgewählt ist, die aus Wasserstoff, einer substituierten oder unsubstituierten Alkylgruppe mit 1 bis 12 Kohlenstoffatomen und einer substituierten oder unsubstituierten Arylgruppe besteht, mit der Maßgabe, dass an jedes Siliciumatom Si¹ zumindest ein Rest R² gebunden ist, der nicht Wasserstoff ist;
Z eine substituierte oder unsubstituierte, aliphatische oder aromatische Gruppe ist;
zur Herstellung einer Isocyanat-Verbindung der allgemeinen Formel I
A³-N=C=O (Formel I),
worin
A³ eine Gruppe der Formel G-V ist: 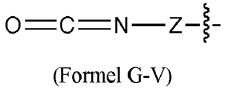
worin Z die in Zusammenhang mit der Verbindung der allgemeinen Formel II angegebenen Bedeutungen hat.

## Claims

1. A method for the preparation of isocyanates, wherein
(i) a first organosilicon compound having at least one silicon atom Si¹ and a unit of formula G-I bound thereto 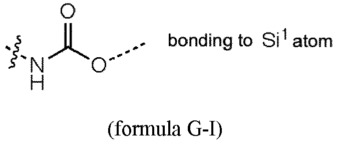 is converted to a third organosilicon compound having a unit of formula G-II 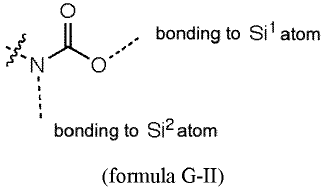 by silylation of the NH group of the unit of formula G-I with a second organosilicon compound having one silicon atom Si²; and
(ii) the third organosilicon compound is reacted to an isocyanate by thermolysis, whereby the unit of formula G-II is converted to an isocyanate group.

2. A method according to claim 1, **characterized in that** the first organosilicon compound is a compound of general formula II 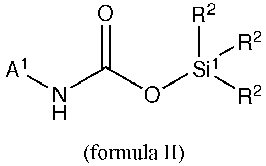 wherein
A¹ is R¹ or a group of general formula G-III: 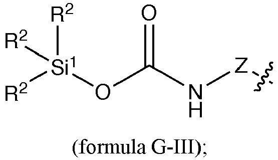
R¹ is a substituted or unsubstituted, aliphatic or aromatic group;
R² at each occurrence each independently is selected from the group consisting of hydrogen, a substituted or unsubstituted alkyl group with 1 to 12 carbon atoms and a substituted or unsubstituted aryl group, with the proviso that at least one residue R² which is not hydrogen is bound to each silicon atom Si¹; and
Z is a substituted or unsubstituted, aliphatic or aromatic group;
the second organosilicon compound is a compound of general formula III 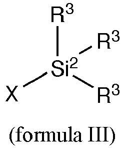
wherein
X is selected from the group consisting of a halogen, -CN, -OCN, -SCN, -N₃, a sulphonate, a carbamate, -O-R⁴, -NR⁷R⁸ or an N-heterocycle;
R³ at each occurrence each independently is selected from the group consisting of hydrogen, a substituted or unsubstituted alkyl group with 1 to 12 carbon atoms and a substituted or unsubstituted aryl group, with the proviso that at least one residue R³ which is not hydrogen is bound to each silicon atom Si²;
R⁴ is -C(O)R⁹ or a group of general formula G-VI 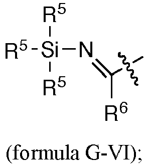
R⁵ at each occurrence each independently is a substituted or unsubstituted alkyl group with 1 to 12 carbon atoms;
R⁶ is a substituted or unsubstituted alkyl group with 1 to 12 carbon atoms;
R⁷ and R⁸ each independently are -C(O)R⁹ or a substituted or unsubstituted alkyl group with 1 to 12 carbon atoms; and
R⁹ is a substituted or unsubstituted alkyl group with 1 to 12 carbon atoms;
and the third organosilicon compound is a compound of general formula IV 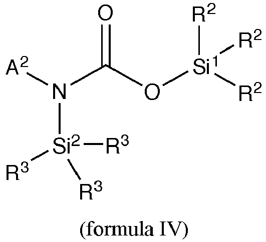
wherein
A² is R¹ or a group of general formula G-IV: 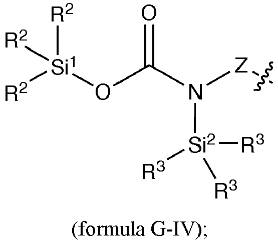
R² at each occurrence and Z have the meanings given in connection with the compound of general formula II; and
R³ at each occurrence has the meanings given in connection with the compound of general formula III;
wherein the compound of general formula II is reacted with the compound of general formula III to the compound of general formula IV and the compound of general formula IV is reacted to an isocyanate compound of general formula I 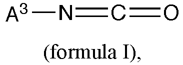
wherein
A³ is R¹ or a group of formula G-V: 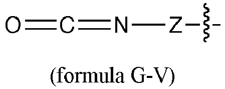
R¹ and Z have the meanings given in connection with the compound of general formula 11;
by thermolysis.

3. The method according to claim 2, **characterized in that** R¹ in the compounds of general formula I, II and IV is selected from the group consisting of a substituted or unsubstituted alkyl group with 1 to 18 carbon atoms, a substituted or unsubstituted heteroalkyl group with 1 to 18 carbon atoms, a substituted or unsubstituted alkenyl group with 1 to 18 carbon atoms, a substituted or unsubstituted alkynyl group with 1 to 18 carbon atoms, a substituted or unsubstituted aryl group and a substituted or unsubstituted heteroaryl group.

4. The method according to claim 2 or claim 3, **characterized in that** R¹ in the compounds of general formula I, II and IV is selected from the group consisting of methyl, ethyl, propyl, butyl, pentyl, hexyl, octyl, and phenyl.

5. The method according to any of claims 2 to 4, **characterized in that** Z in the compounds of general formula I, II and IV is selected from the group consisting of a substituted or unsubstituted alkylene group with 1 to 12 carbon atoms, a substituted or unsubstituted phenylene group, and a substituted or unsubstituted phenylene-bisalkylene group in which each alkylene group at each occurrence each independently is an alkylene group with 1 to 12 carbon atoms.

6. The method according to any of claims 2 to 5, **characterized in that** X in the compound of general formula III is selected from the group consisting of chlorine, a toluene sulphonic acid ester group, a methyl sulphonic acid ester group, and a trifluoromethyl sulphonic acid ester group.

7. The method according to any of claims 2 to 6, **characterized in that** R³ at each occurrence in the compounds of general formula III and IV each independently is a substituted or unsubstituted alkyl group with 1 to 6 carbon atoms or a substituted or unsubstituted phenyl group.

8. The method according to any of claims 2 to 7, **characterized in that** all of R² in the compounds of general formula I, II and IV each are a methyl group and/or all of R³ in the compounds of general formula III and IV each are a methyl group.

9. The method according to any of the preceding claims, **characterized in that** the silylation is carried out at a temperature of 0 to 50°C.

10. The method according to any of the preceding claims, **characterized in that** the silylation is carried out in a non-polar organic solvent in the presence of an auxiliary base.

11. The method according to any of the preceding claims, **characterized in that** the thermolysis is carried out at a temperature of 150 to 400°C.

12. The method according to any of the preceding claims, **characterized in that** the thermolysis is carried out using a temperature profile comprising two or more temperature stages, wherein each temperature stage is applied for a given time period and each of the applied temperature stages is above the previously applied temperature range by 20 to 80°C.

13. The method according to claim 12, **characterized in that** the given period of time is between 1 min and 2 hrs.

14. The method according to any of the preceding claims, **characterized in that** the thermolysis is carried out in the presence of an inert gas.

15. Use of a compound of general formula II 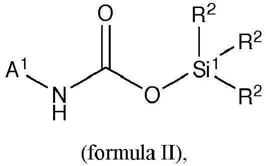 wherein
A' is a group of general formula G-III: 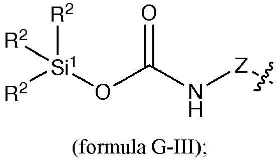
R² at each occurrence each independently is selected from the group consisting of hydrogen, a substituted or unsubstituted alkyl group with 1 to 12 carbon atoms and a substituted or unsubstituted aryl group, with the proviso that at least one residue R² which is not hydrogen is bound to each silicon atom Si¹;
Z is a substituted or unsubstituted, aliphatic or aromatic group;
for the preparation of an isocyanate compound of general formula I
A³-N=C=O (formula I),
wherein
A³ is a group of formula G-V: 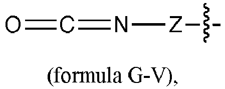
wherein Z has the meanings given in connection with the compound of general formula II.

## Revendications

1. Procédé de fabrication d'isocyanates,
(i) un premier composé organosilicié, qui présente au moins un atome de silicium Si¹ et une unité de formule G-I liée à celui-ci, 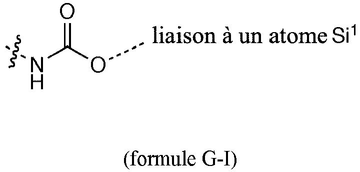 étant transformé par silylation du groupe NH de l'unité de la formule G-I avec un deuxième composé organosilicié, qui présente un atome de silicium Si², en un troisième composé organosilicié, qui présente une unité de la formule G-II, 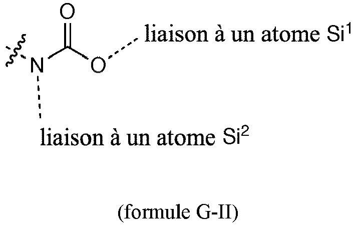 et
(ii) le troisième composé organosilicié étant converti par thermolyse en un isocyanate, l'unité de la formule G-II étant transformée en un groupe isocyanate.

2. Procédé selon la revendication 1, **caractérisé en ce que** le premier composé organosilicié est un composé de la formule générale II 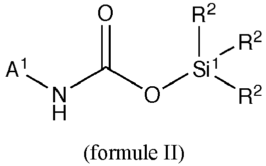 où
A¹ est R¹ ou un groupe de la formule générale G-III : 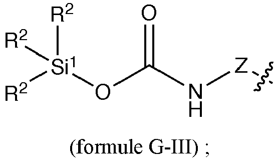
R¹ est un groupe aliphatique ou aromatique, substitué ou non-substitué ;
R² est sélectionné à chaque occurrence de façon indépendante respectivement parmi le groupe, qui est constitué d'hydrogène, d'un groupe alkyle substitué ou non substitué avec 1 à 12 atomes de carbone et d'un groupe aryle substitué ou non substitué, à la condition qu'à chaque atome de silicium Si¹ soit lié au moins un radical R² qui n'est pas l'hydrogène ; et
Z est un groupe aliphatique ou aromatique, substitué ou non-substitué ;
le deuxième composé organosilicié est un composé de la formule générale III 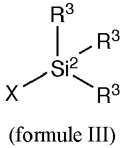
où
X est sélectionné parmi le groupe, qui est constitué d'un halogène, -CN, -OCN, -SCN, -N₃, d'un sulfonate, d'un carbamate, -O-R⁴, -NR⁷R⁸ ou d'un N-hétérocycle ;
R³ est sélectionné à chaque occurrence de façon indépendante respectivement parmi le groupe, qui est constitué d'hydrogène, d'un groupe alkyle substitué ou non substitué avec 1 à 12 atomes de carbone et d'un groupe aryle substitué ou non substitué, à la condition qu'à chaque atome de silicium Si² soit lié au moins un radical R³, qui n'est pas l'hydrogène ;
R⁴ est -C(O)R⁹ ou un groupe de la formule générale G-VI 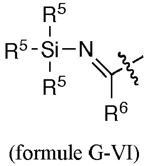
R⁵ est à chaque occurrence de façon indépendante respectivement un groupe alkyle substitué ou non substitué avec 1 à 12 atomes de carbone ;
R⁶ est un groupe alkyle substitué ou non substitué avec 1 à 12 atomes de carbone ;
R⁷ et R⁸ sont de façon indépendante respectivement -C(O)R⁹ ou un groupe alkyle substitué ou non substitué avec 1 à 12 atomes de carbone ; et
R⁹ est un groupe alkyle substitué ou non substitué avec 1 à 12 atomes de carbone ;
et le troisième composé organosilicié est un composé de la formule générale IV 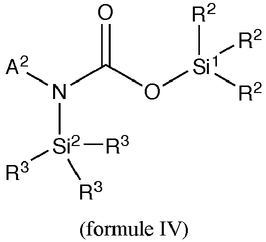
où
A² est R¹ ou un groupe de la formule générale G-IV : 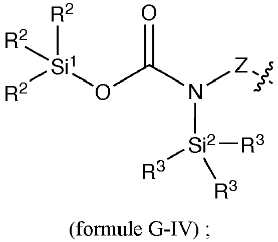
R² à chaque occurrence et Z ont les significations spécifiées en rapport avec le composé de la formule générale II ; et
R³ a à chaque occurrence les significations spécifiées en rapport avec le composé de la formule générale III ;
le composé de la formule générale II avec le composé de la formule générale III étant converti en composé de la formule générale IV et le composé de la formule générale IV étant converti par thermolyse en un composé d'isocyanate de la formule générale I 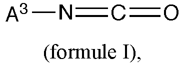
où
A³ est R¹ ou un groupe de la formule G-V: 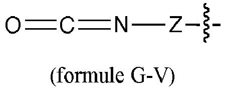
R¹ et Z ont les significations spécifiées en rapport avec le composé de la formule générale II.

3. Procédé selon la revendication 2, **caractérisé en ce que** R¹ dans les composés des formules générales I, II et IV est sélectionné parmi le groupe, qui est constitué d'un groupe alkyle substitué ou non substitué avec 1 à 18 atomes de carbone, d'un groupe hétéroalkyle substitué ou non substitué avec 1 à 18 atomes de carbone, d'un groupe alcényle substitué ou non substitué avec 1 à 18 atomes de carbone, d'un groupe alkinyle substitué ou non substitué avec 1 à 18 atomes de carbone, d'un groupe aryle, substitué ou non substitué et d'un groupe hétéroaryle substitué ou non substitué.

4. Procédé selon la revendication 2 ou la revendication 3, **caractérisé en ce que** R¹ dans les composés des formules générales I, II et IV est sélectionné parmi le groupe, qui est constitué de méthyle, d'éthyle, de propyle, de butyle, de pentyle, d'hexyle, d'octyle et de phényle.

5. Procédé selon l'une des revendications 2 à 4, **caractérisé en ce que** Z dans les composés des formules générales I, II et IV est sélectionné parmi le groupe, qui est constitué d'un groupe alkylène substitué ou non substitué avec 1 à 12 atomes de carbone, d'un groupe phénylène substitué ou non substitué et d'un groupe phénylène-bisalkylène substitué ou non substitué, dans lequel chaque groupe alkylène à chaque occurrence de façon indépendante respectivement est un groupe alkylène avec 1 à 12 atomes de carbone.

6. Procédé selon l'une des revendications 2 à 5, **caractérisé en ce que** X dans le composé de la formule générale III est sélectionné parmi le groupe, qui est constitué de chlore, d'un groupe d'ester d'acide toluènesulfonique, d'un groupe d'ester d'acide méthylsulfonique et d'un groupe d'ester d'acide trifluorométhylsulfonique.

7. Procédé selon l'une des revendications 2 à 6, **caractérisé en ce que** R³ à chaque occurrence dans les composés de la formule générale III et IV de façon indépendante respectivement est un groupe alkyle substitué ou non substitué avec 1 à 6 atomes de carbone ou un groupe phényle substitué ou non substitué.

8. Procédé selon l'une des revendications 2 à 7, **caractérisé en ce que** tous les R² dans les composés des formules générales I, II et IV sont chacun un groupe méthyle et/ou tous les R³ dans les composés des formules générales III et IV sont chacun un groupe méthyle.

9. Procédé selon l'une des revendications précédentes, **caractérisé en ce que** la silylation est effectuée à une température de 0 à 50 °C.

10. Procédé selon l'une des revendications précédentes, **caractérisé en ce que** la silylation est effectuée dans un solvant organique non polaire en présence d'une base auxiliaire.

11. Procédé selon l'une des revendications précédentes, **caractérisé en ce que** la thermolyse est effectuée à une température de 150 à 400 °C.

12. Procédé selon l'une des revendications précédentes, **caractérisé en ce que** la thermolyse est effectuée en appliquant un profil de température, qui comprend deux ou trois niveaux de température, chaque niveau de température étant appliqué à une période prescrite et chaque niveau de température appliqué se situant de 20 à 80 °C au-dessus de la plage de température précédemment appliquée.

13. Procédé selon la revendication 12 **caractérisé en ce que** la période prescrite se situe entre 1 min et 2 h.

14. Procédé selon l'une des revendications précédentes, **caractérisé en ce que** la thermolyse est effectuée en présence d'un gaz inerte.

15. Utilisation d'un composé de la formule générale II 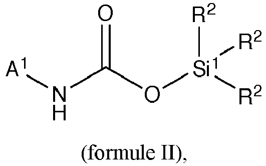 où
A¹ est un groupe de la formule générale G-III : 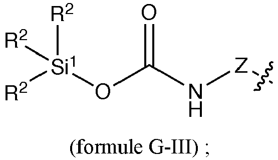
R² est sélectionné à chaque occurrence de façon indépendante respectivement parmi le groupe, qui est constitué d'hydrogène, d'un groupe alkyle, substitué ou non substitué avec 1 à 12 atomes de carbone et d'un groupe aryle, substitué ou non substitué, à la condition qu'à chaque atome de silicium Si¹ soit lié au moins un radical R², qui n'est pas l'hydrogène ;
Z est un groupe aliphatique ou aromatique, substitué ou non-substitué ;
pour la fabrication d'un composé d'isocyanate de la formule générale I
A³-N=C=O (formule I),
où
A³ est un groupe de la formule G-V : 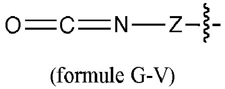
où Z a les significations spécifiées en rapport avec le composé de la formule générale II.
